(19) Europäisches Patentamt
European Patent Office
Office européen des brevets

(11) **EP 4 723 130 A1**

(12) **EUROPEAN PATENT APPLICATION**
published in accordance with Art. 153(4) EPC

(43) Date of publication:
**08.04.2026 Bulletin 2026/15**

(21) Application number: **25760871.1**

(22) Date of filing: **24.02.2025**

(51) International Patent Classification (IPC):
**G16H 70/40** (2018.01)

(52) Cooperative Patent Classification (CPC):
**G06F 18/214; G06F 18/24; G16B 20/30;
G16B 20/50; G16B 50/30; G16H 20/10;
G16H 50/70; G16H 70/40**

(86) International application number:
**PCT/CN2025/078835**

(87) International publication number:
**WO 2025/180330 (04.09.2025 Gazette 2025/36)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **01.03.2024 CN 202410239951**

(71) Applicant: **GeneMind Biosciences Company
Limited
Shenzhen, Guangdong 518023 (CN)**

(72) Inventors:
• **CHEN, Yanxiang
Shenzhen, Guangdong 518023 (CN)**
• **ZHOU, Letian
Shenzhen, Guangdong 518023 (CN)**

(74) Representative: **Schiweck Weinzierl Koch
Patentanwälte Partnerschaft mbB
Ganghoferstraße 68 B
80339 München (DE)**

(54) **DRUG RESISTANCE DATABASE CONSTRUCTION METHOD AND APPARATUS, DRUG RESISTANCE DETECTION METHOD AND APPARATUS, AND DEVICE**

(57) Disclosed are a method and apparatus for constructing a drug resistance database, a drug resistance testing method and apparatus, and a device. The method comprises: acquiring a preset classification object set of a target drug corresponding to each of at least one classification object dimension; acquiring, for each classification object dimension, an object feature set of the target drug separately corresponding to each preset classification object in the preset classification object set of the classification object dimension; screening, based on an object weight and a sample mutation vector set in each object feature set, the preset classification object set, to obtain a target classification object set; and based on at least one target classification object set, constructing in a drug resistance database a standard drug resistance mutation information set corresponding to the target drug, wherein each classification object dimension comprises a gene dimension and/or a mutation site dimension. Embodiments of the present invention improve the efficiency and accuracy of constructing a drug resistance database.

Acquire a preset classification object set of a target drug corresponding to each of at least one classification object dimension — S110

Acquire, for each classification object dimension, an object feature set of the target drug separately corresponding to each preset classification object in the preset classification object set of the classification object dimension — S120

Screen, based on an object weight and a sample mutation vector set in each object feature set, the preset classification object set, to obtain a target classification object set — S130

Based on at least one target classification object set, construct in a drug resistance database a standard drug resistance mutation information set corresponding to the target drug — S140

FIG. 1

## Description

### TECHNICAL FIELD

The present invention relates to the field of bioinformatics, and in particular to a method for constructing a drug resistance database, a drug resistance testing method, apparatus, and a device.

### BACKGROUND ART

If a patient misses a dose of medication, takes medication late, changes medication without authorization, or stops medication without authorization during treatment, a pathogenic strain may develop drug resistance to a therapeutic drug, rendering an original treatment scheme ineffective. A phenotypic resistance test for analyzing the drug resistance of a pathogenic strain often takes several weeks. Waiting for results of the drug resistance test before administering medication will greatly delay treatment of a disease. Therefore, rapid testing of the drug resistance of the pathogenic strain is of positive significance for prevention and control of the disease.

Currently, a relatively commonly used method for testing the drug resistance of a pathogenic strain is to employ gene sequencing means to acquire mutation site information of the pathogenic strain, compare the mutation site information with drug resistance mutation information corresponding to each of a plurality of therapeutic drugs organized in a drug resistance database, and determine, according to comparison results, whether the pathogenic strain is resistant to a certain therapeutic drug.

The described drug resistance testing method places quite high requirements on the drug resistance database. However, current drug resistance databases rely on manual organization, resulting in problems such as untimely updates, incomplete drug resistance mutation information, and inaccurate drug resistance mutation information.

### SUMMARY OF THE INVENTION

Embodiments of the present invention provide a method and apparatus for constructing a drug resistance database, a drug resistance testing method and apparatus, and a device, to solve the problem of conventional drug resistance databases requiring manual organization, thereby improving the efficiency, comprehensiveness, and accuracy of constructing a drug resistance database.

According to an embodiment of the present invention, a method for constructing a drug resistance database is provided, the method comprising:

Acquiring a preset classification object set of a target drug corresponding to each of at least one classification object dimension;

Acquiring, for each classification object dimension, an object feature set of the target drug separately corresponding to each preset classification object in the preset classification object set of the classification object dimension;

Screening, based on an object weight and a sample mutation vector set in each object feature set, the preset classification object set to obtain a target classification object set; and

Based on at least one target classification object set, constructing in a drug resistance database a standard drug resistance mutation information set corresponding to the target drug, where

Each classification object dimension comprises a gene dimension and/or a mutation site dimension, the sample mutation vector set comprises at least two object mutation vectors, each object mutation vector comprises at least one mutation site identifier of a strain sample corresponding to the preset classification object, and the mutation site identifier represents whether reference drug resistance mutation information in a reference drug resistance mutation information set exists in a sample mutation information set corresponding to the strain sample.

According to an embodiment of the present invention, a drug resistance testing method is provided, the method comprising:

Acquiring a mutation information set to be tested for a strain to be tested, where the mutation information set to be tested comprises at least one piece of mutation information to be tested;

Acquiring in a drug resistance database a standard drug resistance mutation information set corresponding to a target drug, where the standard drug resistance mutation information set comprises at least one piece of standard drug resistance mutation information; and

Determining, based on overlapping data corresponding to the mutation information set to be tested and the standard drug resistance mutation information set, a target drug resistance result of the strain to be tested against the target drug, where

The drug resistance database is obtained using the method for constructing a drug resistance database according to any embodiment of the present invention.

According to another embodiment of the present invention, an apparatus for constructing a drug resistance database is provided, the apparatus comprising:

A preset classification object set acquisition module, configured to acquire a preset classification object set of a target drug corresponding to each of at least one classification object dimension;

An object feature set acquisition module, configured to acquire, for each classification object dimension, an object feature set of the target drug separately corresponding to each preset classification object in the preset classification object set of the classification object dimension;

A preset classification object set screening module, configured to screen, based on an object weight and a sample mutation vector set in each object feature set, the preset classification object set, to obtain a target classification object set; and

A drug resistance database construction module, configured to, based on at least one target classification object set, construct in a drug resistance database a standard drug resistance mutation information set corresponding to the target drug, wherein

Each classification object dimension comprises a gene dimension and/or a mutation site dimension, the sample mutation vector set comprises at least two object mutation vectors, each object mutation vector comprises at least one mutation site identifier of a strain sample corresponding to the preset classification object, and the mutation site identifier represents whether reference drug resistance mutation information in a reference drug resistance mutation information set exists in a sample mutation information set corresponding to the strain sample.

According to another embodiment of the present invention, a drug resistance testing apparatus is provided, the apparatus comprising:

A mutation information set to be tested acquisition module, configured to acquire a mutation information set to be tested for a strain to be tested, wherein the mutation information set to be tested comprises at least one piece of mutation information to be tested;

A standard drug resistance mutation information set acquisition module, configured to acquire in a drug resistance database a standard drug resistance mutation information set corresponding to a target drug, wherein the standard drug resistance mutation information set comprises at least one piece of standard drug resistance mutation information; and

A target drug resistance result determination module, configured to determine, based on overlapping data corresponding to the mutation information set to be tested and the standard drug resistance mutation information set, a target drug resistance result of the strain to be tested against the target drug, wherein

The drug resistance database is obtained using the method for constructing a drug resistance database according to any embodiment of the present invention.

According to another embodiment of the present invention, an electronic device is provided, the electronic device comprising:

At least one processor; and

A memory communicatively connected to the at least one processor, where

The memory stores a computer program executable by the at least one processor, and the computer program is executed by the at least one processor to enable the at least one processor to perform the method for constructing a drug resistance database and/or the drug resistance testing method according to any embodiment of the present invention.

According to another embodiment of the present invention, a computer-readable storage medium is provided, where the computer-readable storage medium stores computer instructions configured to, when executed by a processor, cause the processor to implement the method for constructing a drug resistance database and/or the drug resistance testing method according to any embodiment of the present invention.

In the technical solutions of the embodiments of the present invention, the preset classification object set of the target drug corresponding to each of the at least one classification object dimension is acquired; for each classification object dimension, the object feature set of the target drug separately corresponding to each preset classification object in the preset classification object set of the classification object dimension is acquired; the preset classification object set is screened based on the object weight and the sample mutation vector set in each object feature set to obtain the target classification object set; and the standard drug resistance mutation information set corresponding to the target drug is constructed in the drug resistance database based on at least one target classification object set, where each classification object dimension comprises the gene dimension and/or the mutation site dimension. This solves the problem of conventional drug resistance databases requiring manual organization and improves the construction efficiency, comprehensiveness, and accuracy of a drug resistance database.

It should be understood that the content described in this section is not intended to identify key or important features of the embodiments of the present invention, nor is the content described in this section intended to limit the scope of the present invention. Other features of the present invention will become readily apparent from the following description.

## BRIEF DESCRIPTION OF THE DRAWINGS

To more clearly illustrate the technical solutions in embodiments of the present invention, drawings to be used in the description of the embodiments will be briefly described below. Obviously, the drawings in the following description are merely some embodiments of the present invention. For those of ordinary skill in the art, other drawings can further be obtained according to those drawings without the exercise of inventive effort.

FIG. 1 is a flowchart of a method for constructing a drug resistance database provided by an embodiment of the present invention;

FIG. 2 is a flowchart of a method for screening a preset classification object set provided by an embodiment of the present invention;

FIG. 3 is a flowchart of a specific example of a method for constructing a drug resistance database provided by an embodiment of the present invention;

FIG. 4 is a flowchart of another method for constructing a drug resistance database provided by an embodiment of the present invention;

FIG. 5 is a flowchart of another method for screening a preset classification object set provided by an embodiment of the present invention;

FIG. 6 is a flowchart of a drug resistance testing method provided by an embodiment of the present invention;

FIG. 7 is a ROC plot of a drug resistance testing method provided by Embodiment 1 of the present invention for a 10% testing set in Table 1;

FIG. 8 is a ROC plot of a drug resistance testing method provided by Embodiment 2 of the present invention for Table 2;

FIG. 9 is a ROC plot using TB-profile software provided by a comparative example for the 10% test set in Embodiment 1;

FIG. 10 is a schematic structural diagram of an apparatus for constructing a drug resistance database provided by an embodiment of the present invention;

FIG. 11 is a schematic structural diagram of a drug resistance testing apparatus provided by an embodiment of the present invention; and

FIG. 12 is a schematic structural diagram of an electronic device provided by an embodiment of the present invention.

## DESCRIPTION OF THE PREFERRED EMBODIMENTS

To enable those skilled in the art to better understand the solutions of the present invention, technical solutions in embodiments of the present invention will be clearly and completely described below with reference to accompanying drawings in the embodiments of the present invention. Obviously, the embodiments described are merely some embodiments, not all embodiments, of the present invention. Based on the embodiments of the present invention, all other embodiments obtained by those of ordinary skill in the art without the exercise of inventive effort shall fall within the scope of protection of the present invention.

It should be noted that the terms "first," "second," "preset," "target," "reference," etc., in the description, claims, and drawings of the present invention are used to distinguish similar objects and are not necessarily used to describe a specific order or sequence. It should be understood that data used in this way can be interchanged where appropriate so that the embodiments of the present invention described herein can be implemented in a sequence other than those illustrated or described herein. Furthermore, the terms "comprise" and "have" and any variations thereof are intended to cover non-exclusive inclusion. For example, a process, method, system, product, or device that includes a series of steps or units is not necessarily limited to those steps or units that are explicitly listed, but may include other steps or units that are not explicitly listed or that are inherent to that process, method, product, or device.

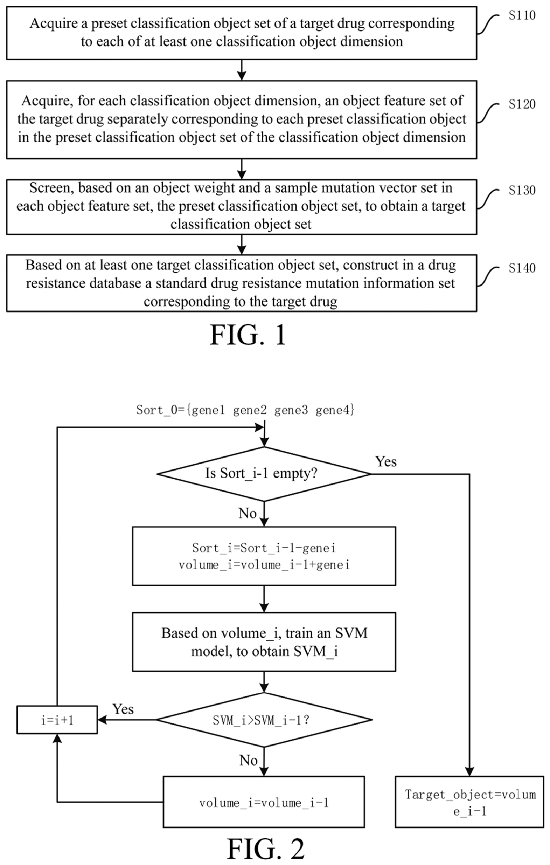

FIG. 1 is a flowchart of a method for constructing a drug resistance database provided by an embodiment of the present invention. The present embodiment is applicable to the construction of a drug resistance database of a pathogenic strain for one or more therapeutic drugs. The method can be performed by an apparatus for constructing a drug resistance database, which can be implemented in the form of hardware and/or software and can be configured in a terminal device. As shown in FIG. 1, the method includes:

S 110. Acquiring a preset classification object set of a target drug corresponding to each of at least one classification object dimension.

Specifically, there is a correlation between the target drug and a strain to which the drug resistance database pertains. That is, the drug resistance database is a database formed with drug resistance information of the specific strain to the target drug acting on that strain. This drug resistance information includes whether the strain is resistant to the target drug, as well as a gene locus, drug resistance mutation site, etc., that may be involved in the drug resistance of the strain to the target drug. For the specific strain, the target drug is a drug that can produce drug activity or at least has produced drug activity when acting on the strain or a disease caused by the strain. For example, assuming that the strain is Mycobacterium tuberculosis, target drugs include, but are not limited to, rifampicin, isoniazid, pyrazinamide, or ethambutol. If the strain is Escherichia coli, target drugs include, but are not limited to, amikacin, piperacillin/tazobactam, or cefoxitin. The selection of the strain and the target drug is not limited herein, and the same can be specifically customized according to actual needs.

Specifically, the classification object dimension refers to an object dimension in which information related to drug resistance of the strain to the target drug is classified, and the preset classification object set refers to a feature set acquired by classifying resistance-related information for a preset classification object dimension. In an embodiment of the present application, the resistance-related information is a reference drug resistance mutation information set, and each classification object dimension may include a resistance gene-based object dimension, which is also referred to herein as a gene dimension. Each classification object dimension may further include an object dimension of a mutation site that affects drug resistance, which is referred to herein as a mutation site dimension. Each classification object dimension may also include both the gene dimension and the mutation site dimension. The reference drug resistance mutation information set includes at least two pieces of reference drug resistance mutation information acquired in advance.

Specifically, the drug resistance mutation information is used to characterize information of a mutation site that exhibits drug resistance to the target drug in nucleic acid sequence data of the strain. Exemplarily, the drug resistance mutation information includes, but is not limited to, information such as a standard base and a mutated base corresponding to the mutation site relative to a standard nucleic acid sequence of the strain, the name of a gene where the mutation site is located, and the location of the mutation site on a genome. The drug resistance mutation information is not limited herein, and can be specifically set according to actual needs. It should be understood that the standard nucleic acid sequence referred to in the embodiments of the present application refers to a nucleic acid sequence of the strain in a genome where a mutation site is located before a drug resistance mutation occurs.

In an optional embodiment, when the classification object dimension is the gene dimension, the preset classification object set is a preset drug resistance gene set. Specifically, the preset drug resistance gene set includes at least two preset drug resistance genes, which are used to characterize genes in the nucleic acid sequence data of the strain that exhibit drug resistance to the target drug. Using the strain being Mycobacterium tuberculosis and the target drug being rifampicin as an example, a preset drug resistance gene set corresponding to rifampicin includes, but is not limited to, the rpoB gene, the katG gene, the embB gene, the inhA gene, etc.

Exemplarily, nucleic acid sequence data corresponding to strain $J$ includes L preset drug resistance genes, each preset drug resistance gene includes one or more drug resistance mutation sites, and each drug resistance mutation site corresponds to a piece of drug resistance mutation information.

In an optional embodiment, when the classification object dimension is the mutation site dimension, the preset classification object set is a preset drug resistance mutation information set. Specifically, the preset drug resistance mutation information set includes at least two pieces of reference drug resistance mutation information in the reference drug resistance mutation information set. The reference drug resistance mutation information set is a set formed by at least some currently known drug resistance mutation information of the strain to be analyzed. Approaches for acquiring the drug resistance mutation information are not limited herein.

In an optional embodiment, the acquiring a preset classification object set of a target drug corresponding to each of at least one classification object dimension includes: using, when the classification object dimension is the mutation site dimension, the reference drug resistance mutation information set as a preset drug resistance mutation information set of the target drug corresponding to the mutation site dimension.

Based on the above embodiments, optionally, when each classification object dimension includes the gene dimension and the mutation site dimension, each preset classification object set includes the preset drug resistance gene set and the reference drug resistance mutation information set. An advantage of this setup is that parallel screening dimensions consisting of the gene dimension and the mutation site dimension can improve the accuracy of the drug resistance database.

In another alternative embodiment, when the classification object dimension is the gene dimension, the target classification object set is a target drug resistance gene set; accordingly, the acquiring a preset classification object set of a target drug corresponding to each of at least one classification object dimension includes: when each classification object dimension includes the gene dimension and the mutation site dimension, based on the target drug resistance gene set corresponding to the gene dimension, performing a filtering operation on the reference drug resistance mutation information set to obtain a preset drug resistance mutation information set of the target drug corresponding to the mutation site dimension.

For example, assuming the target drug resistance gene set includes drug resistance gene A and drug resistance gene B, and the reference drug resistance mutation information set includes three pieces of reference drug resistance mutation information corresponding to drug resistance gene A, five pieces of reference drug resistance mutation information corresponding to drug resistance gene B, and two pieces of reference drug resistance mutation information corresponding to drug resistance gene C, the preset drug resistance mutation information set includes eight pieces of reference drug resistance mutation information in total, including the three pieces of reference drug resistance mutation information corresponding to drug resistance gene A and the five pieces of reference drug resistance mutation information corresponding to drug resistance gene B.

An advantage of this setup is that sequential screening dimensions consisting of the gene dimension and the mutation site dimension can improve the accuracy of the drug resistance database.

S120. Acquiring, for each classification object dimension, an object feature set of the target drug separately corresponding to each preset classification object in the preset classification object set of the classification object dimension.

In the present embodiment, the object feature set includes an object weight and a sample mutation vector set. The sample mutation vector set includes at least two object mutation vectors. Each object mutation vector includes at least one mutation site identifier of a strain sample corresponding to the preset classification object. The mutation site identifier represents whether reference drug resistance mutation information in the reference drug resistance mutation information set exists in a sample mutation information set corresponding to the strain sample.

Specifically, the object weight is used to characterize a degree of influence of a preset classification object on drug resistance classification testing. In an optional embodiment, when the classification object dimension is the gene dimension, the object weight is a gene weight. When the classification object dimension is the mutation site dimension, the object weight is a mutation site weight. The gene weight is used to characterize a degree of influence of a preset drug resistance gene on drug resistance classification testing, and the mutation site weight is used to characterize a degree of influence of reference drug resistance mutation site information on drug resistance classification testing.

Specifically, the sample mutation vector set includes a sample mutation vector of at least one strain sample having a drug drug resistance label of resistant separately corresponding to a preset classification object, as well as a sample mutation vector of at least one strain sample having a drug drug resistance label of unresistant separately corresponding to a preset classification object.

In an optional embodiment, when the classification object dimension is the gene dimension, each object mutation vector is a gene mutation vector. When the classification object dimension is the mutation site dimension, each sample mutation vector is a site mutation vector. Specifically, the gene mutation vector includes a mutation site identifier of at least one piece of reference drug resistance mutation information corresponding to a preset drug resistance gene in the reference drug resistance mutation information set, and the site mutation vector includes a mutation site identifier corresponding to the reference drug resistance mutation information.

Exemplarily, the mutation site identifier may be represented in the form of a number, text, or a graphic. For example, when the mutation site identifier is represented in the form of a number, the mutation site identifier may be 0 or 1. When the mutation site identifier is represented in the form of text, the mutation site identifier may be yes or no. When the mutation site identifier is represented in the form of a graphic, the mutation site identifier may be "O" or "✕". The representation form of the mutation site identifier is not limited herein, and can be specifically customized according to actual needs.

Specifically, the sample mutation information set includes at least one piece of sample mutation information corresponding to the strain sample. The sample mutation information may be reference drug resistance mutation information, or may be other mutation information.

Using the object classification dimension being the gene dimension as an example, assuming that the preset drug resistance gene is drug resistance gene A, the reference drug resistance mutation information set includes reference drug resistance mutation information 1, reference drug resistance mutation information 2, and reference drug resistance mutation information 3 corresponding to drug resistance gene A, and a sample mutation information set corresponding to strain sample I includes reference drug resistance mutation information 1 and reference drug resistance mutation information 2, a gene mutation vector corresponding to strain sample I in a sample mutation vector set corresponding to drug resistance gene A is [1 1 0].

Using the object classification dimension being the mutation site dimension as an example, assuming the preset classification object is reference drug resistance mutation information 1, if the sample mutation information set corresponding to strain sample I includes reference drug resistance mutation information 1, then a site mutation vector corresponding to strain sample I in the sample mutation information set corresponding to reference drug resistance mutation information 1 is [1]. If the sample mutation information corresponding to strain sample I does not include reference drug resistance mutation information 1, then the site mutation vector corresponding to strain sample I in the sample mutation information set corresponding to reference drug resistance mutation information 1 is [0].

S130. Screening, based on the object weight and the sample mutation vector set in each object feature set, the preset classification object set to obtain a target classification object set.

In an optional embodiment, the screening, based on the object weight and the sample mutation vector set in each object feature set, the preset classification object set to obtain a target classification object set includes: adding a preset classification object having the greatest object weight in the preset classification object set as a current classification object to a reference classification object set, and removing the current classification object from the preset classification object set; training, based on a sample mutation vector set separately corresponding to at least one preset classification object in the reference classification object set, a first initial model to obtain a current first target model; acquiring previous first classification performance of a previous first target model in a previous iteration step; iteratively performing, when current first classification performance of the current first target model is better than the previous first classification performance, the step of adding a preset classification object having the greatest object weight in the preset classification object set as a current classification object to the reference classification object set; and using, until the preset classification object set is empty, the reference classification object set as the target classification object set.

Specifically, during the iterative screening process of the preset classification object set, each preset classification object in the preset classification object set is sequentially added to the reference classification object set as the current classification object in descending order of object weight.

Specifically, when the reference classification object set includes one preset classification object, the first initial model is trained based on a sample mutation vector set corresponding to the preset classification object and a drug resistance label of each strain sample separately corresponding to the target drug, to obtain the current first target model. When the reference classification object set includes a plurality of preset classification objects, for each strain sample, sample mutation vectors of the strain sample respectively corresponding to the preset classification objects are merged into a list mutation vector, and the first initial model is trained based on each list mutation vector and the drug resistance labels of each strain sample separately corresponding to the target drug, to obtain the current first target model.

For example, assuming that the reference classification object set includes drug resistance gene A and drug resistance gene B, a sample mutation vector set corresponding to drug resistance gene A includes gene mutation vector [1 0 0] of strain sample I and gene mutation vector [0 1 0] of strain sample II, and a sample mutation vector set corresponding to drug resistance gene B includes gene mutation vector [1 1 0] of strain sample I and gene mutation vector [1 0 1] of strain sample II, list mutation vectors corresponding to strain sample I and strain sample II are [1 0 0 1 1 0] and [0 1 0 1 0 1], respectively.

Exemplarily, the sample mutation vector set or each list mutation vector is classified to obtain a training set, a validation set, and a testing set. The training set is used to train model parameters of the first initial model, the validation set is used to train hyperparameters of the first initial model, and the testing set is used to acquire the current first classification performance of the current first target model.

Exemplarily, a model architecture of the first initial model includes, but is not limited to, ResNet, a Transformer network, a CNN (convolutional neural network), an FCN (fully convolutional network), a DNN (deep neural network), an RNN (recurrent neural network), or an SVM (support vector machine), etc. The model architecture of the first initial model is not limited herein, and can be specifically customized according to actual needs.

Exemplarily, a performance metric used for the first classification performance includes, but is not limited to, at least one of accuracy, precision, an F1 score, an error rate, a ROC curve, AUC (area under curve), etc. The performance metric used for the first classification performance is not limited herein, and can be specifically customized according to actual needs.

Specifically, in the first iteration step, the previous first classification performance corresponding to the first iteration step may be set to a preset numerical value or the greatest object weight in each object feature set. Exemplarily, the preset numerical value may be 0. The preset numerical value is not limited herein, and can be specifically customized according to actual needs.

In an optional embodiment, the method further includes: setting, if current accuracy of the current first target model is greater than previous accuracy of a previous first target model, the current first classification performance to be better than the previous first classification performance; or setting, if the current accuracy of the current first target model is less than or equal to the previous accuracy of the previous first target model, the current first classification performance to be not better than the previous first classification performance.

In another alternative embodiment, the method further includes: acquiring a difference in accuracy corresponding to the current accuracy of the current first target model and the previous accuracy of the previous first target model; and setting, if the difference in accuracy is less than a preset difference threshold, the current first classification performance to be better than the previous first classification performance; or setting, if the difference in accuracy is greater than or equal to the preset difference threshold, the current first classification performance to be not better than the previous first classification performance.

Exemplarily, the preset difference threshold may be 0.01. The preset difference threshold is not limited herein, and can be specifically customized according to actual needs.

Based on the above embodiments, optionally, the screening, based on the object weight and the sample mutation vector set in each object feature set, the preset classification object set to obtain a target classification object set further includes: removing, when the current first classification performance of the current first target model is not better than the previous first classification performance, the current classification object from the reference classification object set; and iteratively performing the step of adding a preset classification object having the greatest object weight in the preset classification object set as a current classification object to the reference classification object set.

FIG. 2 is a flowchart of a screening method for a preset classification object set provided by an embodiment of the present invention. FIG. 2 uses the preset classification objects being preset drug resistance genes as an example. The preset classification object set includes four drug resistance genes, denoted as Sort_0={gene1 gene2 gene3 gene4}. In the i-th iteration step, it is determined whether a preset classification object set Sort_i-1 corresponding to the (i-1)-th iteration step is empty. If yes, a reference classification object set Volume_i-1 obtained in the (i-1)-th iteration step is used as a target classification object set Target_object. If not, the i-th drug resistance gene genei, sorted in descending order of gene weight in a preset classification object set Sort_0, is added to the reference classification object set Volume_i-1 to obtain a reference classification object set Volume_i, and the drug resistance gene genei is removed from the preset classification object set Sort_i-1 to obtain a preset classification object set Sort_i. Based on a sample mutation vector set separately corresponding to each drug resistance gene in the reference classification object set Volume_i and a drug drug resistance label of each strain sample, an SVM model is trained to obtain a current first target model SVM_i. It is determined whether current first classification performance of the current first target model SVM_i is better than current first classification performance of a previous first target model SVM_i-1. If yes, i=i+1 is set and a next iteration step is started. If not, the drug resistance gene genei is removed from the reference classification object set Volume_1. That is, the reference classification object set Volume_i-1 is used as the reference classification object set Volume_i, i=i+1 is set, and the next iteration step is started.

S140. Based on at least one target classification object set, construct in the drug resistance database a standard drug resistance mutation information set corresponding to the target drug.

In an optional embodiment, constructing in the drug resistance database, based on at least one target classification object set, a standard drug resistance mutation information set corresponding to the target drug includes: acquiring, when each classification object dimension includes only the gene dimension, for each target drug resistance gene in the target drug resistance gene set corresponding to the gene dimension, a gene drug resistance mutation information set consisting of at least one piece of target drug resistance mutation information corresponding to the target drug resistance gene in the reference drug resistance mutation information set; and using each gene drug resistance mutation information set as the standard drug resistance mutation information set corresponding to the target drug in the drug resistance database.

In another optional embodiment, the constructing in the drug resistance database, based on at least one target classification object set, a standard drug resistance mutation information set corresponding to the target drug includes: using, when each classification object dimension includes only the mutation site dimension, a target drug resistance mutation information set corresponding to the mutation site dimension as the standard drug resistance mutation information set corresponding to the target drug in the drug resistance database.

In another optional embodiment, when the classification object dimension is the gene dimension, the target classification object set is a target drug resistance gene set, and when the classification object dimension is the mutation site dimension, the target classification object set is a target drug resistance mutation information set; accordingly, the constructing in the drug resistance database, based on at least one target classification object set, a standard drug resistance mutation information set corresponding to the target drug includes: acquiring, when each classification object dimension includes the gene dimension and the mutation site dimension having a parallel relationship, for each target drug resistance gene in the target drug resistance gene set corresponding to the gene dimension, a gene drug resistance mutation information set consisting of at least one piece of target drug resistance mutation information corresponding to the target drug resistance gene in the reference drug resistance mutation information set; and constructing in the drug resistance database, based on at least one gene drug resistance mutation information set and a target drug resistance mutation information set corresponding to the mutation site dimension, the standard drug resistance mutation information set corresponding to the target drug.

Specifically, a merging operation is performed on each gene drug resistance mutation information set, and a union operation is performed on the merged set and the target drug resistance mutation information set to obtain the standard drug resistance mutation information set corresponding to the target drug in the drug resistance database.

In another optional embodiment, the constructing in the drug resistance database, based on at least one target classification object set, a standard drug resistance mutation information set corresponding to the target drug includes: using, when each classification object dimension includes the gene dimension and mutation site dimension having a serial relationship, a target drug resistance mutation information set corresponding to the mutation site dimension as the standard drug resistance mutation information set corresponding to the target drug in the drug resistance database.

Based on the above embodiments, optionally, after the constructing in the drug resistance database, based on at least one target classification object set, a standard drug resistance mutation information set corresponding to the target drug, the method further includes: performing a union operation on the standard drug resistance mutation information set corresponding to the target drug in the drug resistance database and a conventional drug resistance mutation information set corresponding to the target drug in a conventional drug resistance database to obtain a merged drug resistance database.

An advantage of this setup is that the conventional drug resistance database is expanded and filtered, thereby further improving the comprehensiveness and accuracy of the drug resistance database.

Based on the above embodiments, optionally, the standard drug resistance mutation information set further includes a mutation score separately corresponding to at least one piece of standard drug resistance mutation information. Specifically, when the standard drug resistance mutation information is reference drug resistance mutation information, the mutation score is a mutation site weight. When the standard drug resistance mutation information is conventional drug resistance mutation information, the mutation score is a credibility rating provided by the conventional drug resistance database.

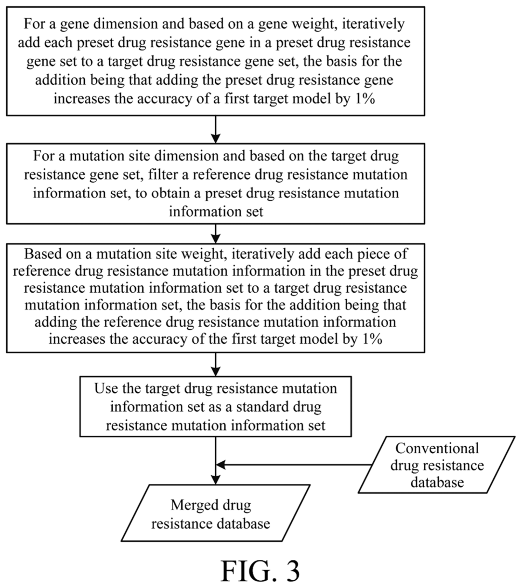

FIG. 3 is a flowchart of a specific example of a method for constructing a drug resistance database provided by an embodiment of the present invention. Specifically, for the gene dimension, based on a sorting method in descending order of gene weight, each preset drug resistance gene in the preset drug resistance gene set is iteratively added to the target drug resistance gene set. The basis for the addition is to add the preset drug resistance gene to the target drug resistance gene set, so that accuracy of the first target model obtained by training based on the target drug resistance gene set after the addition is increased by 1% compared with accuracy of the first target model obtained by training based on the target drug resistance gene set before the addition. For the mutation site dimension, based on the target drug resistance gene set, the reference drug resistance mutation information set is filtered to obtain a preset drug resistance mutation information set. Based on a sorting method in descending order of mutation site weight, each piece of reference drug resistance mutation information in the preset drug resistance mutation information set is iteratively added to the target drug resistance mutation information set. The basis for the addition is to add the reference drug resistance mutation information to the target drug resistance mutation information set, so that the accuracy of the first target model obtained by training based on the target drug resistance mutation information set after the addition is increased by 1% compared with the accuracy of the first target model obtained by training based on the target drug resistance mutation information set before the addition. The target drug resistance mutation information set is used as the standard drug resistance mutation information set, and a union operation is performed on the standard drug resistance mutation information set and the conventional drug resistance mutation information set in the conventional drug resistance database to obtain the merged drug resistance database.

In the technical solution of the present embodiment, the preset classification object set of the target drug corresponding to each of the at least one classification object dimension is acquired; for each classification object dimension, the object feature set of the target drug separately corresponding to each preset classification object in the preset classification object set of the classification object dimension is acquired; the preset classification object set is screened based on the object weight and the sample mutation vector set in each object feature set to obtain the target classification object set; and the standard drug resistance mutation information set corresponding to the target drug is constructed in the drug resistance database based on at least one target classification object set, where each classification object dimension includes the gene dimension and/or the mutation site dimension. This solves the problem of conventional drug resistance databases requiring manual organization and improves the construction efficiency, comprehensiveness, and accuracy of a drug resistance database.

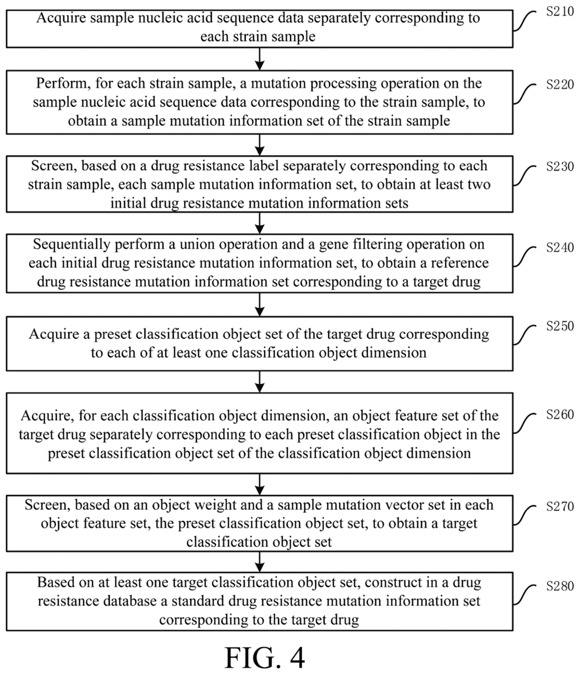

FIG. 4 is a flowchart of another method for constructing a drug resistance database provided by an embodiment of the present invention. The present embodiment further refines the method for constructing a drug resistance database in the above embodiments. As shown in FIG. 4, the method includes:

S210. Acquiring sample nucleic acid sequence data separately corresponding to each strain sample.

In an optional embodiment, the acquiring sample nucleic acid sequence data separately corresponding to each strain sample includes: acquiring from a nucleic acid sequence database sample nucleic acid sequence data separately corresponding to at least two strain samples.

In another optional embodiment, the acquiring sample nucleic acid sequence data separately corresponding to each strain sample includes: performing, for each strain sample, a sequencing operation on a strain nucleic acid of the strain sample to obtain the sample nucleic acid sequence data. Exemplarily, sequencing tools used in the sequencing operation include, but are not limited to, Illumina MiSeq tools and PacBio sequel II tools. The sequencing tools used in the sequencing operation are not limited herein, and can be specifically customized according to actual needs.

Exemplarily, the nucleic acid sequence data may be whole genome nucleic acid sequence data or targeted nucleic acid sequence data such as tNGS. The data type of the nucleic acid sequence data is not limited herein, and can be specifically customized according to actual needs.

Based on the above embodiments, optionally, after the acquiring sample nucleic acid sequence data separately corresponding to each strain sample, the method further includes: separately performing quality control processing on each piece of sample nucleic acid sequence data. Exemplarily, the quality control processing includes, but is not limited to, removing linkers, filtering low-quality sequences, filtering excessively short sequences, etc. Quality control tools used in the quality control processing may be FastP tools, Trimmomatic tools, or FastQC tools. The quality control tools used in the quality control processing are not limited herein, and can be specifically customized according to actual needs.

S220. Performing, for each strain sample, a mutation processing operation on the sample nucleic acid sequence data corresponding to the strain sample to obtain a sample mutation information set of the strain sample.

In an optional embodiment, the mutation processing operation includes an alignment operation, a sorting operation, a deduplication operation, a gene mutation site identification operation, a filtering operation, and an annotation operation performed in sequence.

Specifically, the alignment operation refers to comparing the sample nucleic acid sequence data with standard nucleic acid sequence data of the strain to obtain alignment result data. The alignment result data includes information such as a starting position, an alignment direction, an alignment score, and a mismatch condition of the sample nucleic acid sequence data aligned to the standard nucleic acid sequence data. Exemplarily, alignment tools used in the alignment operation may be bowtie2 alignment tools, BWA-MEM tools, BWA-MEM2 tools, SNAP tools, or Minimap2 tools. The alignment tools used in the alignment operation are not limited herein, and can be specifically customized according to actual needs.

Specifically, the sorting operation refers to sorting the alignment result data according to alignment coordinates in the alignment result data to obtain sorted alignment result data. Exemplarily, sorting tools used in the sorting operation may be SAMtools tools or Sambamba tools. The sorting tools used in the sorting operation are not limited herein, and can be specifically customized according to actual needs.

Specifically, the deduplication operation refers to performing PCR deduplication processing on the sorted alignment result data. Exemplarily, duplicated redundant parts of the same alignment results that are aligned to the same alignment coordinate in the sorted alignment result data are removed to obtain alignment result data that has undergone deduplication. Exemplarily, deduplication tools used in the deduplication operation may be GATK tools, Sambamba tools, SAMtools tools, or Picard tools. The deduplication tools used in the deduplication operation are not limited herein, and can be specifically customized according to actual needs.

Specifically, the gene mutation site identification operation refers to identifying mutation sites and performing hard filtering on the alignment result data that has undergone deduplication to generate mutation site identification result data. Exemplarily, gene mutation site identification tools used in the gene mutation site identification operation may be GATK tools, VarScan tools, BCFtools tools, or Platypus tools. The gene mutation site identification tools used in the gene mutation site identification operation are not limited herein, and can be specifically customized according to actual needs.

Specifically, the filtering operation refers to filtering the mutation site identification result data to remove mutations in highly mutable PE/PPE gene families, duplicated regions, and mobile elements, to obtain filtered mutation site identification result data. Exemplarily, filtering tools used in the filtering operation may be VCFtools tools. The filtering tools used in the filtering operation are not limited herein, and can be specifically customized according to actual needs.

Specifically, the annotation operation refers to using annotation tools to perform mutation type annotation and remove synonymous mutations from the filtered mutation site identification result data, to obtain a sample mutation information set corresponding to the strain sample. Exemplarily, annotation tools used in the annotation operation may be ANNOVAR tools, SnpEff tools, or Ensembl VEP tools. The annotation tools used in the annotation operation are not limited herein, and can be specifically customized according to actual needs.

S230. Screening, based on a drug resistance label separately corresponding to each strain sample, each sample mutation information set to obtain at least two initial drug resistance mutation information sets.

Specifically, the drug resistance label is either resistant or unresistant, and the initial drug resistance mutation information set represents a sample mutation information set corresponding to a strain sample having a drug resistance label of resistant.

S240. Performing a union operation and a gene filtering operation on each initial drug resistance mutation information set in sequence, to obtain a reference drug resistance mutation information set corresponding to the target drug.

The gene filtering operation refers to filtering, according to a preset drug resistance gene set, a union drug resistance information set obtained after the union operation, to obtain the reference drug resistance mutation information set. Specifically, for each piece of drug resistance mutation information in the union drug resistance information set obtained after the union operation, if a gene where the drug resistance mutation information is located does not exist in the preset drug resistance gene set, the drug resistance mutation information is removed from the union drug resistance information set.

S250. Acquiring a preset classification object set of the target drug corresponding to each of at least one classification object dimension.

S250 in the present embodiment corresponds to or is similar to S110 shown in FIG. 1 of the above embodiment, and will not be repeated in the present embodiment.

S260. Acquiring, for each classification object dimension, an object feature set of the target drug separately corresponding to each preset classification object in the preset classification object set of the classification object dimension.

In an optional embodiment, when the classification object dimension is the gene dimension, the preset classification object set is a preset drug resistance gene set, and the object weight is a gene weight; accordingly, the acquiring an object feature set of the target drug separately corresponding to each preset classification object in the preset classification object set of the classification object dimension includes: acquiring a sample mutation vector set of the target drug separately corresponding to each preset drug resistance gene in the preset drug resistance gene set of the gene dimension; training, for each preset drug resistance gene, based on the sample mutation vector set corresponding to the preset drug resistance gene, a third initial model to obtain a trained third target model; determining, based on third classification performance corresponding to the third target model, a gene weight corresponding to the preset drug resistance gene; and adding a sample mutation vector set and a gene weight corresponding to the preset drug resistance gene to an object feature set corresponding to the preset drug resistance gene.

Specifically, a performance parameter value corresponding to the third classification performance is used as the gene weight corresponding to the preset drug resistance gene. Exemplarily, a model architecture of the third initial model includes, but is not limited to, a Transformer network, a CNN, an FCN, a residual network, a DNN, an RNN, or an SVM, etc. A performance metric used for the third classification performance includes, but is not limited to, at least one of accuracy, precision, F1 score, error rate, ROC curve, AUC, etc. The model architecture of the third initial model and the performance metric used for the third classification performance are not limited herein, and can be specifically customized according to actual needs.

In an optional embodiment, when the classification object dimension is the mutation site dimension, the preset classification object set is a preset drug resistance mutation information set, the object weight is a mutation site weight, and the object mutation vector is a site mutation vector; accordingly, the acquiring an object feature set of the target drug separately corresponding to each preset classification object in the preset classification object set of the classification object dimension includes: acquiring sample mutation features respectively corresponding to at least two strain samples, where each sample mutation feature includes a site mutation vector of the strain sample corresponding to each piece of reference drug resistance mutation information in the reference drug resistance mutation information set; training, based on each sample mutation feature, a fourth initial model to obtain a trained fourth target model; using, for each piece of reference drug resistance mutation information in the preset drug resistance mutation information set, a model weight corresponding to the reference drug resistance mutation information in the fourth target model as a mutation site weight; and adding the sample mutation vector set and the mutation site weight corresponding to the reference drug resistance mutation information to an object feature set corresponding to the reference drug resistance mutation information.

Specifically, for each piece of reference drug resistance mutation information in the reference drug resistance mutation information set, if the sample mutation information set includes the reference drug resistance mutation information, a site mutation vector corresponding to the reference drug resistance mutation information in a sample mutation feature is set to 1; if the sample mutation information set does not include the reference drug resistance mutation information, the site mutation vector corresponding to the reference drug resistance mutation information in the sample mutation feature is set to 0.

In an optional embodiment, a model architecture of the fourth initial model is an SVM model. The SVM model is a binary classification model, and a basic model thereof is defined as a linear classifier having the largest margin in a feature space, and a learning strategy thereof is to maximize the margin, which can ultimately be transformed into solving a convex quadratic programming problem. In an embodiment, the sample mutation feature is a feature array consisting of 0 and 1, where 1 represents that the reference drug resistance mutation site information is detected in the strain sample, and 0 represents that the reference drug resistance mutation site information is not detected in the strain sample. Exemplarily, a sample mutation feature of the $i$-th strain sample may be represented using $X^i$, and a drug resistance label may be represented using $Y^i$.

A goal of the SVM model is to find a maximally separating hyperplane that divides input data into two classes. The linear hyperplane used is defined as Y = ($W^T X + b$), in which $W$ includes a model weight corresponding to each site mutation vector in the sample mutation feature. Exemplarily, $W = [w_1, w_2, ... , w_N]$, where $N$ represents the number of site mutation vectors, and $b$ represents an intercept.Considering that some strain samples near boundaries may not be well separated by the hyperplane, regularization processing is used in the SVM model, and the binary classification problem is defined as: under the premise that a condition $Y^i(W^T X^i + b) \geq 1 - \xi^i$, $\xi^i \geq 1$, $i = 1,2, ... , N$ holds, solve $W$ and $b$ such that a formula $min_{w,b} \frac{1}{2}W^2 + C \sum_i^N \xi^i$ holds.

Exemplarily, a performance metric used for the fourth classification performance includes, but is not limited to, at least one of accuracy, precision, F1 score, error rate, ROC curve, AUC, etc. The model architecture of the fourth initial model and the performance metric used for the fourth classification performance are not limited herein, and can be specifically customized according to actual needs.

S270. Screening, based on an object weight and a sample mutation vector set in each object feature set, the preset classification object set to obtain a target classification object set.

In an optional embodiment, S270 corresponds to or is similar to S130 shown in FIG. 1 of the above embodiment, and will not be repeated in the present embodiment.

In another optional embodiment, the screening, based on an object weight and a sample mutation vector set in each object feature set, the preset classification object set to obtain a target classification object set includes: adding a preset classification object having the greatest object weight in the preset classification object set to a current screened classification object set; adding at least one preset classification object that does not exist in the current screened classification object set to the current screened classification object set to obtain at least one current reference classification object set; and screening, based on each current reference classification object set and at least two sample mutation vector sets, the preset classification object set to obtain the target classification object set.

For example, assuming that the preset classification object set includes drug resistance gene A, drug resistance gene B, drug resistance gene C, and drug resistance gene D, and the current screening classification object set is [drug resistance gene A drug resistance gene B]. Correspondingly, each reference classification object includes drug resistance gene C and drug resistance gene D, in which case each current reference classification object set includes [drug resistance gene A drug resistance gene B drug resistance gene C] and [drug resistance gene A drug resistance gene B drug resistance gene D].

Based on the above embodiments, optionally, the screening, based on each current reference classification object set and at least two sample mutation vector sets, the preset classification object set to obtain the target classification object set includes: training, for each current reference classification object set, based on a sample mutation vector set separately corresponding to at least one preset classification object in the current reference classification object set, a second initial model to obtain a current second target model; acquiring previous second classification performance of a previous second target model corresponding to a previous screened classification object set in a previous iteration step; determining, based on the previous second classification performance and current second classification performance separately corresponding to at least one current second target model, a next screened classification object set, and using the next screened classification object set as the current screened classification object set; iteratively performing the step of adding at least one preset classification object that does not exist in the current screened classification object set to the current screened classification object set to obtain at least one current reference classification object set; and using, until each current second classification performance is not better than the previous second classification performance, the current screened classification object set as the target classification object set.

Specifically, a training method for the second initial model is the same as or similar to the training method for the first initial model in the above embodiments, and will not be repeated herein in the present embodiment.

Specifically, the determining, based on the previous second classification performance and current second classification performance separately corresponding to at least one current second target model, a next screened classification object set includes: using the best current second classification performance as reference second classification performance; and using, if the reference second classification performance is better than previous second classification performance, a current reference classification object set corresponding to the reference second classification performance as the next screened classification object set.

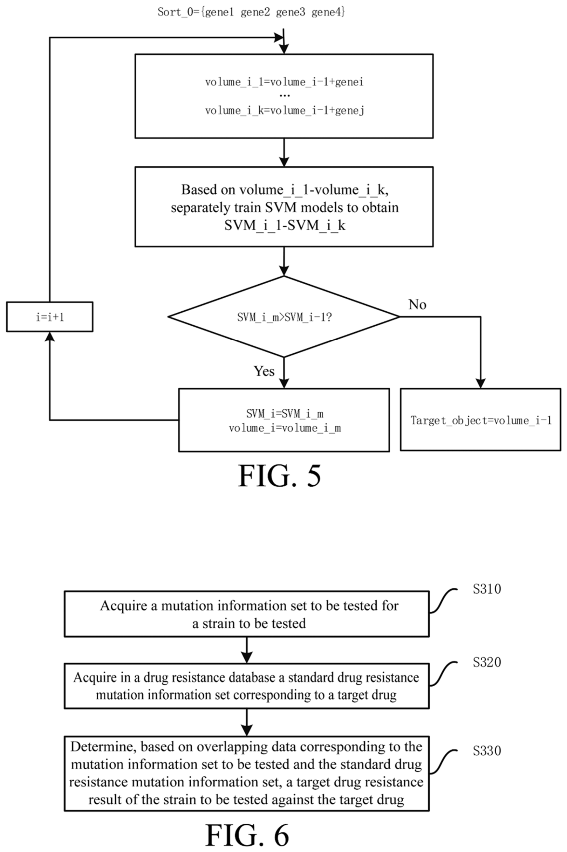

FIG. 5 is a flowchart of another method for screening a preset classification object set provided by an embodiment of the present invention. FIG. 5 uses the preset classification object set being a preset drug resistance gene as an example. The preset classification object set includes four drug resistance genes, denoted as Sort_0={gene1, gene2, gene3, gene4}. In the i-th iteration step, k reference drug resistance genes, which are genei to genej, respectively, are each added to a screened classification object set Volume_i-1 obtained in the (i-1)-th iteration step, resulting in k reference classification object sets, which are Volume_i_l to Volume_i_k, respectively. For each reference classification object set, an SVM model is trained based on at least one sample mutation vector set corresponding to the reference classification object set and the drug resistance label of each strain sample to obtain a current second target model. A second target model with the best current second classification performance in k current second target models, which are SVM_i_1 to SVM_i_k, respectively, is acquired, and it is determined whether reference second classification performance of the reference second target model SVM_i_m is better than previous second classification performance of a previous second target model SVM_i-1. If not, the screened classification object set Volume_i-1 obtained in the (i-1)-th iteration step is used as a target classification object set Target_object. If yes, the reference second target model SVM_i_m is used as a second target model SVM_i corresponding to the i-th iteration step, and a current reference classification object set Volume_i_m corresponding to the reference second target model SVM_i_m is used as a screened classification object set Volume_i corresponding to the i-th iteration step, and i=i+1 is set and a next iteration step is started.

S280. Constructing in the drug resistance database, based on at least one target classification object set, a standard drug resistance mutation information set corresponding to the target drug.

S280 in the present embodiment corresponds to or is similar to S140 shown in FIG. 1 of the above embodiment, and will not be repeated in the present embodiment.

In the technical solution of the embodiment, the sample nucleic acid sequence data corresponding to each strain sample is acquired; for each strain sample, the mutation processing operation is performed on the sample nucleic acid sequence data corresponding to the strain sample to obtain the sample mutation information set of the strain sample; each sample mutation information set is screened based on the drug resistance label separately corresponding to each strain sample to obtain the at least two initial drug resistance mutation information sets; and the union operation and the gene filtering operation are sequentially performed on each initial drug resistance mutation information set to obtain the reference drug resistance mutation information set corresponding to the target drug. This solves the problem of acquiring the reference drug resistance mutation information set, and improves the comprehensiveness of the reference drug resistance mutation information set, thereby further improving the construction efficiency, comprehensiveness, and accuracy of the drug resistance database.

FIG. 6 is a flowchart of a drug resistance testing method provided by an embodiment of the present invention. the present embodiment is applicable to testing of drug resistance of a pathogenic strain against a certain therapeutic drug. The method can be performed by a drug resistance testing apparatus, which may be implemented in the form of hardware and/or software and may be configured in a terminal device. As shown in FIG. 6, the method includes:

S310. Acquiring a mutation information set to be tested for a strain to be tested.

In the present embodiment, the mutation information set to be tested includes at least one piece of mutation information to be tested. A method for acquiring the mutation information set to be tested is the same as or similar to the method for acquiring the sample mutation information set in the above embodiments, and will not be repeated in the present embodiment.

S320. Acquiring in a drug resistance database a standard drug resistance mutation information set corresponding to a target drug.

The drug resistance database used in the present embodiment is obtained through the method for constructing a drug resistance database provided in any of the above embodiments, and will not be repeated herein.

In the present embodiment, the standard drug resistance mutation information set includes at least one piece of standard drug resistance mutation information.

S330. Determining, based on overlapping data corresponding to the mutation information set to be tested and the standard drug resistance mutation information set, a target drug resistance result of the strain to be tested against the target drug.

In an optional embodiment, the determining, based on overlapping data corresponding to the mutation information set to be tested and the standard drug resistance mutation information set, a target drug resistance result of the strain to be tested against the target drug includes: acquiring the number of overlaps between the mutation information set to be tested and the standard drug resistance mutation information set; and setting, when the number of overlaps exceeds a preset number threshold, a target drug resistance result of the strain to be tested against the target drug as resistant; or setting, when the number of overlaps is less than or equal to the preset number threshold, the target drug resistance result of the strain to be tested against the target drug as unresistant.

In another optional embodiment, the determining, based on overlapping data corresponding to the mutation information set to be tested and the standard drug resistance mutation information set, a target drug resistance result of the strain to be tested against the target drug includes: acquiring the number of overlaps between the mutation information set to be tested and a standard drug resistance mutation information set, and using a ratio of the number of overlaps to the number of standard mutations corresponding to the standard drug resistance mutation information set as an overlap rate; and setting, when the overlap rate is greater than an overlap rate threshold, the target drug resistance result of the strain to be tested against the target drug as resistant; or setting, when the overlap rate is less than or equal to the overlap rate threshold, the target drug resistance result of the strain to be tested against the target drug as unresistant.

In another optional embodiment, the standard drug resistance mutation information set further includes a mutation score separately corresponding to each piece of standard drug resistance mutation information, and the overlap data includes an overlap rate; accordingly, the determining, based on overlapping data corresponding to the mutation information set to be tested and the standard drug resistance mutation information set, a target drug resistance result of the strain to be tested against the target drug includes: performing a union operation on the mutation information set to be tested and the standard drug resistance mutation information set to obtain an overlapping mutation information set; determining, based on the mutation score separately corresponding to each piece of standard drug resistance mutation information in the overlapping mutation information set, an overlap rate of the strain to be tested; and determining, based on the overlap rate, the target drug resistance result of the strain to be tested against the target drug.

Specifically, a statistical value of at least one mutation score corresponding to the overlapping mutation information set is used as the overlap rate of the strain to be tested. Exemplarily, the statistical value includes, but is not limited to, a sum, a maximum value, a minimum value, an average value, a median value, etc. The statistical value is not limited herein, and can be specifically customized according to actual needs.

Specifically, when the overlap rate is greater than the overlap rate threshold, the target drug resistance result of the strain to be tested against the target drug is set as resistant; when the overlap rate is less than or equal to the overlap rate threshold, the target drug resistance result of the strain to be tested against the target drug is set as unresistant.

Based on the above embodiments, optionally, before acquiring in a drug resistance database a standard drug resistance mutation information set corresponding to a target drug, the method further includes: acquiring sample mutation features respectively corresponding to at least two strain samples, wherein each sample mutation feature includes a site mutation vector of the strain sample separately corresponding to each piece of reference drug resistance mutation information in the reference drug resistance mutation information set; separately training, based on each sample mutation feature, at least two fifth initial models to obtain at least two trained fifth target models; determining, based on the mutation information set to be tested and the reference drug resistance mutation information set, a mutation feature to be tested corresponding to the strain to be tested; separately inputting the mutation feature to be tested into the at least two fifth target models to obtain predicted drug resistance results respectively outputted by the respective fifth target models; and using, when at least two predicted drug resistance results are the same, the predicted drug resistance result as the target drug resistance result of the strain to be tested against the target drug.

Specifically, model architectures of various fifth initial models are different. In an optional embodiment, each fifth initial model includes an SVM model and a CNN network.

The CNN network is a new type of artificial neural network method that combines artificial neural network and deep learning technology, and features global training that combines local receptive regions, hierarchical structure, feature extraction, and classification processes, and therefore has a relatively accurate recognition capability for local parts of images. Compared to other image recognition algorithms, CNN networks use less preprocessing time, which means significantly shorter learning time and less data to learn free parameters, thus reducing memory requirements for network operation and allowing for construction of more powerful neural networks.

In the present embodiment of the present invention, sample mutation features are folded into an N*N feature matrix. For sample mutation features that are less than N*N, zeros are padded at the feature positions to generate a feature vector similar to an image storage format, facilitating subsequent convolution operations. The CNN network constructs convolutional layers to calculate convolutional values on the feature matrix. Each convolutional layer includes convolution calculations and max pooling operations. Calculation results are then passed to a next convolutional layer until the last convolutional layer completes calculation. After processing by a fully connected layer, the result is passed to a final output layer. The output layer uses a softmax activation function to convert probability values calculated by the fully connected layer into two probability distributions in a range [0, 1], corresponding to probability values of positive and negative classes, respectively.

Exemplarily, sample mutation features are divided into a training set, a testing set, and a validation set to train the fifth initial model. A loss function used during the training process may be a cross-entropy loss function.

Based on the above embodiments, the method further includes: performing, when at least two predicted drug resistance results are different, the step of acquiring in a drug resistance database a standard drug resistance mutation information set corresponding to the target drug.

Advantages of this setup are that, in one aspect, multiple deep learning networks provide dual verification of the target drug resistance result, and in another aspect, the deep learning network and the drug resistance database enable dual prediction of the target drug resistance result. Both aspects contribute to further improving the accuracy of the target drug resistance result.

In the technical solution of the embodiment, the mutation information set to be tested for the strain to be tested is acquired, where the mutation information set to be tested includes at least one piece of mutation information to be tested; the standard drug resistance mutation information set corresponding to the target drug in the drug resistance database is acquired, where the standard drug resistance mutation information set includes at least one piece of standard drug resistance mutation information; and the target drug resistance result of the strain to be tested against the target drug is determined based on the overlapping data corresponding to the mutation information set to be tested and the standard drug resistance mutation information set. This solves the problem of poor testing performance of drug resistance testing based on conventional drug resistance databases, improves the accuracy of the target drug resistance result, and provides data support for subsequent medical tasks.

The following description is provided in conjunction with specific embodiments.

Embodiment 1

Whole genome sequencing data of MTB strains with drug susceptibility test results for four first-line antibacterial drugs, isoniacinamide (INH), rifampicin (RIF), ethambutol (EMB), and pyrazinamide (PZA), were used as raw sample data for training a predictive model of drug resistance of a Mycobacterium tuberculosis (MTB) strain for the drugs. Specifically, this example compiled WGS (whole genome sequencing) data from an NCBI- SRA database original sample data for the present invention. For brevity, sequence numbers obtained from sequencing sequences of resistant and unresistant samples in the NCBI database corresponding to each drug will not be shown.

Specifically, referring to step S140 above, drug resistance mutation site database information was acquired based on the original sample data; corresponding sample nucleic acid sequence data was acquired based on the drug resistance mutation site database information; and the sample nucleic acid sequence data was allocated in a ratio of 8:1:1 to obtain a training set, a validation set, and a testing set. the 80% training set and the 10% validation set were used to train a convolutional neural network model and an SVM model and to construct a drug resistance database. The drug resistance testing method provided in the present embodiment of the present invention was used to determine testing performance for drug resistance testing of the 10% testing set in Table 1. Results are shown in Table 1 below.

Table 1

| Target drug | TP | TN | FN | FP | Sensitivity | Specificity | Accuracy |
|---|---|---|---|---|---|---|---|
| Ethambutol | 210 | 195 | 6 | 23 | 0.97 | 0.89 | 0.93 |
| Isoniazid | 179 | 185 | 5 | 1 | 0.97 | 0.99 | 0.98 |
| Pyrazinamide | 122 | 117 | 14 | 21 | 0.9 | 0.85 | 0.87 |
| Rifampicin | 205 | 211 | 8 | 3 | 0.96 | 0.99 | 0.97 |

TP represents the number of positive samples correctly identified, TN represents the number of negative samples correctly identified, FN represents the number of positive samples incorrectly identified, and FP represents the number of negative samples incorrectly identified.

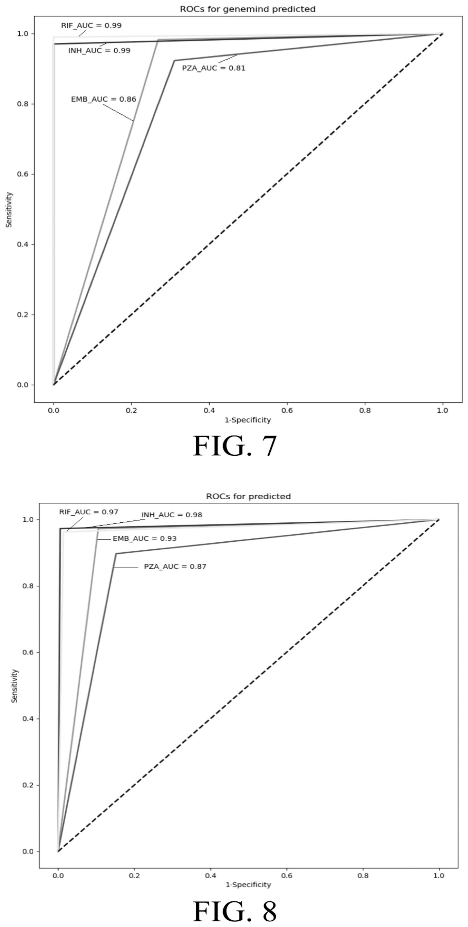

FIG. 7 is a ROC plot of the drug resistance testing method according to Embodiment 1 of the present invention for the 10% testing set in Table 1. Specifically, in FIG. 7, the horizontal axis represents a false positive rate, i.e., 1-specificity, and the vertical axis represents sensitivity. The curves from left to right are ROC curves corresponding to isoniazid, rifampicin, ethambutol, and pyrazinamide, respectively. According to FIG. 7, AUCs corresponding to isoniazid, rifampin, ethambutol, and pyrazinamide were 0.98, 0.97, 0.93, and 0.87, respectively.

Embodiment 2

The present embodiment is basically the same as Embodiment 1, except that original sample data used originated from nucleic acid sequence data respectively corresponding to 424 Mycobacterium tuberculosis samples and four therapeutic drugs in the article: Chen X, He G, Wang S, et al. Evaluation of Whole-Genome Sequence Method to Diagnose Resistance of 13 Anti-tuberculosis Drugs and Characterize drug resistance Genes in Clinical Multi-Resistance Mycobacterium tuberculosis Isolates From China[J]. Other, 2019.DOI:10.3389/fmicb.2019.01741, where the sample nucleic acid sequence data is whole genome nucleic acid sequence data, with the last digit indicating a drug resistance label. For the sake of brevity, the sample nucleic acid sequence data and labels corresponding to each drug are not shown.

Testing performance results of the drug resistance testing method provided in the embodiment of the present invention are shown in Table 2 below. Specifically, the convolutional neural network model, SVM model, and drug resistance database used in the embodiment corresponding to Table 2 were obtained according to the 80% training set and the 10% validation set in Table 1.

Table 2

| Target drug | TP | TN | FN | FP | Sensitivity | Specificity | Accuracy |
|---|---|---|---|---|---|---|---|
| Ethambutol | 57 | 38 | 1 | 14 | 0.98 | 0.73 | 0.86 |
| Isoniazid | 98 | 9 | 3 | 0 | 0.97 | 1 | 0.97 |
| Pyrazinamide | 60 | 31 | 5 | 14 | 0.92 | 0.69 | 0.83 |
| Rifampicin | 99 | 10 | 1 | 0 | 0.99 | 1 | 0.99 |

FIG. 8 is a ROC plot of the drug resistance testing method according to Embodiment 2 of the present invention for the 10% testing set. Specifically, the horizontal axis in FIG. 8 represents a false positive rate, i.e., 1-specificity, and the vertical axis represents sensitivity. The curves from left to right are ROC curves corresponding to rifampicin, isoniazid, ethambutol and pyrazinamide, respectively. According to FIG. 8, AUCs corresponding to isoniazid, rifampicin, ethambutol and pyrazinamide were 0.99, 0.99, 0.86 and 0.81, respectively.

Comparative Example

Drug resistance performance for the 10% testing set in Embodiment 1 was tested using TB-profile software, and results are shown in Table 5 below.

Table 3

| Target drug | TP | TN | FN | FP | Sensitivity | Specificity | Accuracy |
|---|---|---|---|---|---|---|---|
| Ethambutol | 202 | 172 | 14 | 46 | 0.94 | 0.79 | 0.86 |
| Isoniazid | 179 | 185 | 5 | 1 | 0.97 | 0.99 | 0.98 |
| Pyrazinamide | 116 | 113 | 21 | 18 | 0.85 | 0.86 | 0.85 |
| Rifampicin | 205 | 211 | 8 | 3 | 0.96 | 0.99 | 0.97 |

As can be seen from Tables 1 and 3 above, the drug resistance testing method provided in the present embodiment of the present invention achieved basically the same testing results for isoniazid and rifampicin resistance monitoring as compared to the TB-profile software, and showed a significant improvement in the testing performance results for ethambutol and pyrazinamide resistance testing. It is evident that the resistance testing method provided in the present embodiment of the present invention has good accuracy.

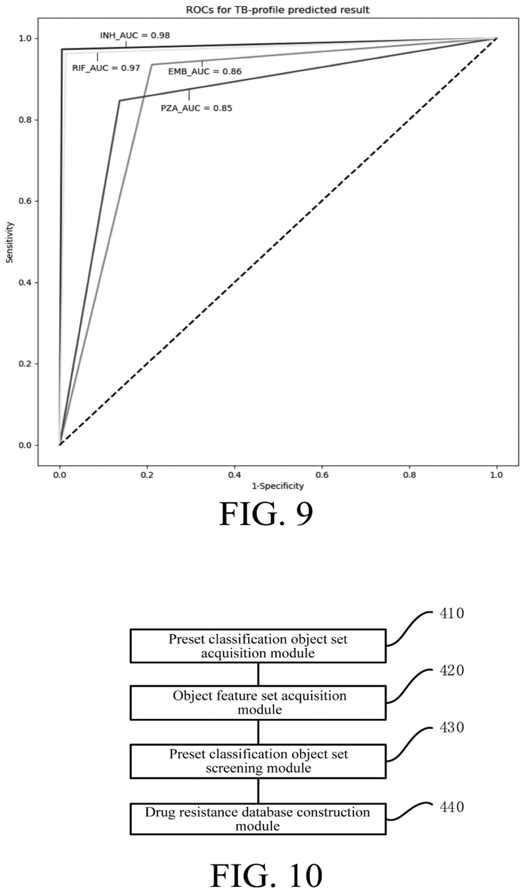

FIG. 9 shows ROC curves for drug resistance testing using the drug resistance testing method in the comparative example. Specifically, the horizontal axis in FIG. 9 represents a false positive rate, i.e., 1-specificity, and the vertical axis represents sensitivity. The curves from left to right are ROC curves for isoniazid, rifampicin, pyrazinamide, and ethambutol, respectively. According to FIG. 9, AUCs for isoniazid, rifampicin, pyrazinamide, and ethambutol were 0.98, 0.97, 0.85, and 0.86, respectively.

As can be seen from FIG. 7 and FIG. 9 above, the drug resistance testing method provided in the present embodiment of the present invention has a significant improvement in AUC performance for ethambutol and pyrazinamide as compared to TB-profile software.

The following are embodiments of an apparatus for constructing a drug resistance database provided in the embodiments of the present invention. The apparatus belongs to the same inventive concept as the method for constructing a drug resistance database in the above embodiments. For details not described in detail in the embodiments of the apparatus for constructing a drug resistance database, reference may be made to the content for the method for constructing a drug resistance database in the above embodiments.

FIG. 10 is a schematic diagram of an apparatus for constructing a drug resistance database provided by an embodiment of the present invention. As shown in FIG. 10, the apparatus includes: a preset classification object set acquisition module 410, an object feature set acquisition module 420, a preset classification object set screening module 430, and a drug resistance database construction module 440.

The preset classification object set acquisition module 410 is configured to acquire a preset classification object set of a target drug corresponding to each of at least one classification object dimension.

The object feature set acquisition module 420 is configured to acquire, for each classification object dimension, an object feature set of the target drug separately corresponding to each preset classification object in the preset classification object set of the classification object dimension.

The preset classification object set screening module 430 is configured to screen, based on an object weight and a sample mutation vector set in each object feature set, the preset classification object set to obtain a target classification object set.

The drug resistance database construction module 440 is configured to construct in the drug resistance database, based on at least one target classification object set, a standard drug resistance mutation information set corresponding to the target drug.

The classification object dimension includes a gene dimension and/or a mutation site dimension, the sample mutation vector set includes at least two object mutation vectors, the object mutation vector includes at least one mutation site identifier of a strain sample corresponding to the preset classification object, and the mutation site identifier represents whether reference drug resistance mutation information in a reference drug resistance mutation information set exists in a sample mutation information set corresponding to the strain sample.

In the technical solution of the present embodiment, the preset classification object set of the target drug corresponding to each of the at least one classification object dimension is acquired; for each classification object dimension, the object feature set of the target drug separately corresponding to each preset classification object in the preset classification object set of the classification object dimension is acquired; the preset classification object set is screened based on the object weight and the sample mutation vector set in each object feature set to obtain the target classification object set; and the standard drug resistance mutation information set corresponding to the target drug is constructed in the drug resistance database based on at least one target classification object set, where the classification object dimension includes the gene dimension and/or the mutation site dimension. This solves the problem of conventional drug resistance databases requiring manual organization and improves the construction efficiency, comprehensiveness, and accuracy of a drug resistance database.

In an optional embodiment, the preset classification object set screening module 430 includes:

A first preset classification object set screening unit, configured to add a preset classification object having the greatest object weight in the preset classification object set as a current classification object to a reference classification object set, and remove the current classification object from the preset classification object set;

Train, based on a sample mutation vector set separately corresponding to at least one preset classification object in the reference classification object set, a first initial model to obtain a current first target model;

Acquiring previous first classification performance of a previous first target model in a previous iteration step;

Iteratively performing, when current first classification performance of the current first target model is better than the previous first classification performance, the step of adding a preset classification object having the greatest object weight in the preset classification object set as a current classification object to the reference classification object set; and

Using the reference classification object set as the target classification object set until the preset classification object set is empty.

In an optional embodiment, the first preset classification object set screening unit is further configured to:

Remove, when the current first classification performance of the current first target model is not better than the previous first classification performance, the current classification object from the reference classification object set; and

Iteratively performing the step of adding a preset classification object having the greatest object weight in the preset classification object set as a current classification object to the reference classification object set.

In an optional embodiment, the preset classification object set screening module 430 includes:

A second preset classification object set screening unit, configured to add a preset classification object having the greatest object weight in the preset classification object set to a current screened classification object set;

Add at least one preset classification object that does not exist in the current screened classification object set to the current screened classification object set to obtain at least one current reference classification object set; and

Screen, based on each current reference classification object set and at least two sample mutation vector sets, the preset classification object set to obtain the target classification object set.

In an optional embodiment, the second preset classification object set screening unit is specifically configured to:

Train, for each current reference classification object set, based on a sample mutation vector set separately corresponding to at least one preset classification object in the current reference classification object set, a second initial model to obtain a current second target model;

Acquire previous second classification performance of a previous second target model corresponding to a previous screened classification object set in a previous iteration step;

Determine, based on the previous second classification performance and current second classification performance separately corresponding to at least one current second target model, a next screened classification object set, and use the next screened classification object set as the current screened classification object set;

Iteratively performing the step of adding at least one preset classification object that does not exist in the current screened classification object set to the current screened classification object set to obtain at least one current reference classification object set; and

Using, until each current second classification performance is not better than the previous second classification performance, the current screened classification object set as the target classification object set.

In an optional embodiment, when the classification object dimension is the gene dimension, the target classification object set is a target drug resistance gene set;
Accordingly, the preset classification object set acquisition module 410 is specifically configured to:
Perform, when the classification object dimension includes the gene dimension and the mutation site dimension, based on the target drug resistance gene set corresponding to the gene dimension, a filtering operation on the reference drug resistance mutation information set to obtain a preset drug resistance mutation information set of the target drug corresponding to the mutation site dimension.
In an optional embodiment, when the classification object dimension is the gene dimension, the target classification object set is a target drug resistance gene set, and when the classification object dimension is the mutation site dimension, the target classification object set is a target drug resistance mutation information set;
Accordingly, the drug resistance database construction module 440 is specifically configured to:

Acquiring, when each classification object dimension includes the gene dimension and the mutation site dimension having a parallel relationship, for each target drug resistance gene in the target drug resistance gene set corresponding to the gene dimension, a gene drug resistance mutation information set consisting of at least one piece of target drug resistance mutation information corresponding to the target drug resistance gene in the reference drug resistance mutation information set; and

Constructing in the drug resistance database, based on at least one gene drug resistance mutation information set and a target drug resistance mutation information set corresponding to the mutation site dimension, the standard drug resistance mutation information set corresponding to the target drug.

In an optional embodiment, the apparatus further includes:

A reference drug resistance mutation information set determination module, configured to acquire sample nucleic acid sequence data separately corresponding to each strain sample;

Perform, for each strain sample, a mutation processing operation on the sample nucleic acid sequence data corresponding to the strain sample to obtain a sample mutation information set of the strain sample;

Screen, based on a drug resistance label separately corresponding to each strain sample, each sample mutation information set to obtain at least two initial drug resistance mutation information sets; and

Sequentially perform a union operation and a gene filtering operation on each initial drug resistance mutation information set, to obtain the reference drug resistance mutation information set corresponding to the target drug.

In an optional embodiment, when the classification object dimension is the gene dimension, the preset classification object set is a preset drug resistance gene set, and the object weight is a gene weight;

Accordingly, the object feature set acquisition module 420 includes:

A first object feature set acquiring unit, configured to acquire a sample mutation vector set of the target drug separately corresponding to each preset drug resistance gene in the preset drug resistance gene set of the gene dimension;

Train, for each preset drug resistance gene, based on the sample mutation vector set corresponding to the preset drug resistance gene, a third initial model to obtain a trained third target model;

Determine, based on third classification performance corresponding to the third target model, a gene weight corresponding to the preset drug resistance gene; and

Add a sample mutation vector set and a gene weight corresponding to the preset drug resistance gene to an object feature set corresponding to the preset drug resistance gene.

In an optional embodiment, when the classification object dimension is the mutation site dimension, the preset classification object set is a preset drug resistance mutation information set, the object weight is a mutation site weight, and the object mutation vector is a site mutation vector;
Accordingly, the object feature set acquisition module 420 includes:

A second object feature set acquiring unit, configured to acquire sample mutation features respectively corresponding to at least two strain samples, where the sample mutation feature includes a site mutation vector of the strain sample corresponding to each piece of reference drug resistance mutation information in the reference drug resistance mutation information set;

Train, based on each sample mutation feature, a fourth initial model to obtain a trained fourth target model;

Use, for each piece of reference drug resistance mutation information in the preset drug resistance mutation information set, a model weight corresponding to the reference drug resistance mutation information in the fourth target model as a mutation site weight; and

Add the sample mutation vector set and the mutation site weight corresponding to the reference drug resistance mutation information to an object feature set corresponding to the reference drug resistance mutation information.

The apparatus for constructing a drug resistance database provided by the present embodiment of the present invention can perform the method for constructing a drug resistance database provided in any embodiment of the present invention, and has corresponding functional modules and beneficial effects of performing the method.
The following are embodiments of a drug resistance testing apparatus provided in the embodiments of the present invention. The apparatus belongs to the same inventive concept as the drug resistance testing method in the above embodiments. For details not described in detail in the embodiments of the drug resistance testing apparatus, reference may be made to the content for the drug resistance testing method in the above embodiments.

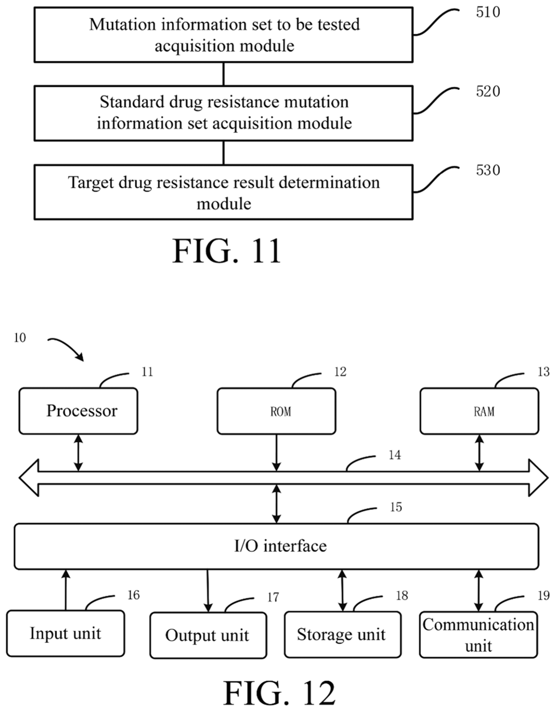

FIG. 11 is a schematic structural diagram of a drug resistance testing apparatus provided by an embodiment of the present invention. As shown in FIG. 11, the apparatus includes: a mutation information set to be tested acquisition module 510, a standard drug resistance mutation information set acquisition module 520, and a target drug resistance result determination module 530.

The mutation information set to be tested acquisition module 510 is configured to acquire a mutation information set to be tested for a strain to be tested. The mutation information set to be tested includes at least one piece of mutation information to be tested.

The standard drug resistance mutation information set acquisition module 520 is configured to acquire in a drug resistance database a standard drug resistance mutation information set corresponding to a target drug. The standard drug resistance mutation information set includes at least one piece of standard drug resistance mutation information.

The target drug resistance result determination module 530 is configured to determine, based on overlapping data corresponding to the mutation information set to be tested and the standard drug resistance mutation information set, a target drug resistance result of the strain to be tested against the target drug.

The drug resistance database is obtained using the method for constructing a drug resistance database provided in any of the above embodiments.

In the technical solution of the embodiment, the mutation information set to be tested for the strain to be tested is acquired, where the mutation information set to be tested includes at least one piece of mutation information to be tested; the standard drug resistance mutation information set corresponding to the target drug is acquired in the drug resistance database, where the standard drug resistance mutation information set includes at least one piece of standard drug resistance mutation information; and the target drug resistance result of the strain to be tested against the target drug is determined based on the overlapping data corresponding to the mutation information set to be tested and the standard drug resistance mutation information set. This solves the problem of poor testing performance of drug resistance testing based on conventional drug resistance databases, improves the accuracy of the target drug resistance result, and provides data support for subsequent medical tasks.

In an optional embodiment, the standard drug resistance mutation information set further includes a mutation score separately corresponding to each piece of standard drug resistance mutation information, and the overlapping data includes an overlap rate;

Accordingly, the target drug resistance result determination module 530 is specifically configured to:

Perform a union operation on the mutation information set to be tested and the standard drug resistance mutation information set, to obtain an overlapping mutation information set;

Determine, based on the mutation score separately corresponding to each piece of standard drug resistance mutation information in the overlapping mutation information set, an overlap rate of the strain to be tested; and

Determine, based on the overlap rate, the target drug resistance result of the strain to be tested against the target drug.

In an optional embodiment, the apparatus further includes:

A predicted drug resistance result determination module, configured to, before acquiring in a drug resistance database a standard drug resistance mutation information set corresponding to a target drug, acquire mutation features respectively corresponding to at least two strain samples, where each sample mutation feature includes a site mutation vector of the strain sample separately corresponding to each piece of reference drug resistance mutation information in the reference drug resistance mutation information set;

Separately train, based on each sample mutation feature, at least two fifth initial models to obtain at least two trained fifth target models;

Determine, based on the mutation information set to be tested and the reference drug resistance mutation information set, a mutation feature to be tested corresponding to the strain to be tested;

Separately input the mutation feature to be tested into the at least two fifth target models to obtain predicted drug resistance results respectively outputted by the respective fifth target models; and

Using, when at least two predicted drug resistance results are the same, the predicted drug resistance result as the target drug resistance result of the strain to be tested against the target drug.

The drug resistance testing apparatus provided by the present embodiment of the present invention can perform the drug resistance testing method provided in any embodiment of the present invention, and has corresponding functional modules and beneficial effects of performing the method.

FIG. 12 is a schematic structural diagram of an electronic device provided by an embodiment of the present invention. The electronic device 10 is intended to represent various forms of digital computers, such as a laptop computer, a desktop computer, a workstation, a server, a blade server, a mainframe computer, and other suitable computers. The electronic device can also refer to various forms of mobile devices, such as a personal digital assistant, a cellular phone, a smartphone, a wearable device (such as a helmet, glasses, a watch, etc.) and other similar computing devices. The components shown herein, connections and relationships thereof, and functions thereof are merely examples and are not intended to limit the implementation of the present invention described and/or claimed herein.

As shown in FIG. 12, the electronic device 10 includes at least one processor 11 and a memory, such as a read-only memory (ROM) 12 or a random access memory (RAM) 13, which is communicatively connected to the at least one processor 11. The memory stores a computer program that can be executed by the at least one processor 11. The processor 11 can perform various appropriate actions and processes according to a computer program stored in the read-only memory (ROM) 12 or a computer program loaded from the storage unit 18 into the random access memory (RAM) 13. The RAM 13 can further store various programs and data required for the operation of the electronic device 10. The processor 11, the ROM 12, and the RAM 13 are interconnected via a bus 14. An input/output (I/O) interface 15 is also connected to the bus 14.

A plurality of components in the electronic device 10 are connected to the I/O interface 15, including: an input unit 16, such as a keyboard, a mouse, etc.; an output unit 17, such as various types of displays, speakers, etc.; a storage unit 18, such as a disk, an optical disk, etc.; and a communication unit 19, such as a network card, a modem, a wireless communication transceiver, etc. The communication unit 19 allows the electronic device 10 to exchange information or data with other devices over computer networks such as the Internet and/or various telecommunications networks.

The processor 11 can be a variety of general-purpose and/or special-purpose processing components with processing and computing capabilities. Examples of the processors 11 include, but are not limited to, a central processing unit (CPU), a graphics processing unit (GPU), various dedicated artificial intelligence (AI) computing chips, various processors that run machine learning model algorithms, a digital signal processor (DSP), and any suitable processor, controller, microcontroller, etc. The processor 11 performs the various methods and processes described above, such as the method for constructing a drug resistance database and/or the drug resistance testing method provided in the above embodiments.

In some embodiments, the method for constructing a drug resistance database and/or the drug resistance testing method provided in the above embodiments can be implemented as a computer program, which is tangibly contained in a computer-readable storage medium, such as a storage unit 18. In some embodiments, part or all of the computer program may be loaded and/or installed on the electronic device 10 via the ROM 12 and/or the communication unit 19. When the computer program is loaded into the RAM 13 and performed by the processor 11, one or more steps of the method for constructing a drug resistance database and/or the drug resistance testing method described above can be performed. Alternatively, in other embodiments, the processor 11 may be configured by any other suitable means (e.g., by means of firmware) to perform the method for constructing a drug resistance database and/or the drug resistance testing method.

Various implementations of the systems and techniques described above herein can be implemented in the following systems or combinations thereof: a digital electronic circuit system, an integrated circuit system, a field programmable gate array (FPGA), an application-specific integrated circuits (ASIC), an application-specific standard part (ASSP), a system on chip (SOC), a complex programmable logic device (CPLDs), computer hardware, firmware, software, and/or combinations thereof. These various implementation methods may include: implementation in one or more computer programs, which can be performed and/or interpreted on a programmable system including at least one programmable processor, which may be a dedicated or general-purpose programmable processor, capable of receiving data and instructions from a storage system, at least one input apparatus, and at least one output apparatus, and transmitting data and instructions to the storage system, the at least one input apparatus, and the at least one output apparatus.

The computer program for the method of constructing a drug resistance database and/or the drug resistance testing method for implementing the present invention can be written in any combination of one or more programming languages. These computer programs can be provided to a processor of a general-purpose computer, a special-purpose computer, or another programmable data processing device, such that when the computer program is performed by the processor, functions/operations specified in the flowcharts and/or block diagrams are performed. The computer program may be executed entirely on a machine, partially on a machine, or as a standalone software package, partially on a machine and partially on a remote machine, or entirely on a remote machine or server.

In the context of the present application, a computer-readable storage medium can be a tangible medium that may include or store a computer program for use by or in conjunction with an instruction execution system, apparatus, or device. The computer-readable storage medium may include, but is not limited to, electronic, magnetic, optical, electromagnetic, infrared, or semiconductor systems, apparatuses, or devices, or any suitable combinations thereof. Alternatively, a computer-readable storage medium may be a machine-readable storage medium. Examples of the machine-readable storage medium include a portable computer disk, hard disk, random access memory (RAM), a read-only memory (ROM), an erasable programmable read-only memory (EPROM), a flash memory, an optical fiber, a compact disc read-only memory (CD-ROM), an optical storage device, a magnetic storage device, or any suitable combination of the foregoing.

To provide interaction with users, the systems and technologies described herein can be implemented on a terminal device, which has: a display device used to display information to users (e.g., cathode ray tube (CRT) or liquid crystal display (LCD) monitors); and keyboard and pointing apparatuses (e.g., a mouse or trackball), via which a user provides an input to the terminal device. Other types of apparatuses can also provide interaction with the user. For example, feedback provided to the user may be any form of sensory feedback (e.g., visual feedback, auditory feedback, or tactile feedback), and may receive input from the user in any form (including voice input, speech input, or tactile input).

The systems and technologies described herein can be implemented in a computing system that includes back-end components (e.g., as a data server), or a computing system that includes middleware components (e.g., an application server), or a computing system that includes a front-end component (e.g., a user computer with a graphical user interface or a web browser through which a user can interact with the implementations of the systems and technologies described herein), or a computing system that includes any combination of such back-end components, middleware components, or front-end components. The components of the system can be interconnected through digital data communication (e.g., communication networks) in any form or medium. Examples of the communication network include: a local area network (LAN), a wide area network (WAN), a blockchain network, and the Internet.

A computing system may include a client and a server. The client and the server are typically located far apart and usually interact through a communication network. A client-server relationship is created by computer programs running on the respective computers and having a client-server relationship with each other. The server can be a cloud server, also known as a cloud computing server or cloud host, which is a host product in a cloud computing service system, which solves shortcomings of conventional physical hosts and virtual private servers (VPS) services, such as high management difficulty and weak business scalability.

It should be understood that the various forms of procedures shown above can be used to reorder, add, or delete steps. For example, the steps described in the present invention can be performed in parallel, sequentially, or in different orders, provided that the desired result of the technical solutions of the present invention can be achieved, and no limitation is imposed herein.

The specific embodiments described above do not constitute a limitation to the scope of protection of the present invention. Those skilled in the art should understand that various modifications, combinations, sub-combinations, and substitutions can be made depending on design requirements and other factors. Any modifications, equivalent substitutions, and improvements made within the spirit and principles of the present invention shall be included within the scope of protection of the present invention.

**Claims**

1. A method for constructing a drug resistance database, **characterized by** comprising:

   acquiring a preset classification object set of a target drug corresponding to each of at least one classification object dimension;
   acquiring, for each classification object dimension, an object feature set of the target drug separately corresponding to each preset classification object in the preset classification object set of the classification object dimension;
   screening, based on an object weight and a sample mutation vector set in each object feature set, the preset classification object set, to obtain a target classification object set; and
   based on at least one target classification object set, constructing in a drug resistance database a standard drug resistance mutation information set corresponding to the target drug, wherein
   each classification object dimension comprises a gene dimension and/or a mutation site dimension, the sample mutation vector set comprises at least two object mutation vectors, each object mutation vector comprises at least one mutation site identifier of a strain sample corresponding to the preset classification object, and the mutation site identifier represents whether reference drug resistance mutation information in a reference drug resistance mutation information set exists in a sample mutation information set corresponding to the strain sample.

2. The method according to claim 1, wherein the screening, based on an object weight and a sample mutation vector set in each object feature set, the preset classification object set, to obtain a target classification object set, comprises:

   adding a preset classification object having the greatest object weight in the preset classification object set to a reference classification object set as a current classification object, and removing the current classification object from the preset classification object set;
   training, based on a sample mutation vector set corresponding to each of at least one preset classification object in the reference classification object set, a first initial model to obtain a current first target model;

acquiring previous first classification performance of a previous first target model in a previous iteration step;

when current first classification performance of the current first target model is better than the previous first classification performance, iteratively performing the step of adding a preset classification object having the greatest object weight in the preset classification object set to the reference classification object set as a current classification object; and

using the reference classification object set as the target classification object set until the preset classification object set is empty.

3. The method according to claim 2, wherein the screening, based on an object weight and a sample mutation vector set in each object feature set, the preset classification object set to obtain a target classification object set further comprises:

removing the current classification object from the reference classification object set when the current first classification performance of the current first target model is not better than the previous first classification performance; and

iteratively performing the step of adding a preset classification object having the greatest object weight in the preset classification object set to the reference classification object set as a current classification object.

4. The method according to claim 1, wherein the screening, based on an object weight and a sample mutation vector set in each object feature set, the preset classification object set to obtain a target classification object set comprises:

adding a preset classification object having the greatest object weight in the preset classification object set to a current screened classification object set;

adding at least one preset classification object that does not exist in the current screened classification object set to the current screened classification object set, to obtain at least one current reference classification object set; and

screening, based on each current reference classification object set and at least two sample mutation vector sets, the preset classification object set, to obtain the target classification object set.

5. The method according to claim 4, wherein the screening, based on each current reference classification object set and at least two sample mutation vector sets, the preset classification object set to obtain the target classification object set comprises:

training, for each current reference classification object set, based on a sample mutation vector set corresponding to each of at least one preset classification object in the current reference classification object set, a second initial model to obtain a current second target model;

acquiring previous second classification performance of a previous second target model corresponding to a previous screened classification object set in a previous iteration step;

determining, based on the previous second classification performance and current second classification performance separately corresponding to at least one current second target model, a next screened classification object set, and using the next screened classification object set as the current screened classification object set;

iteratively performing the step of adding at least one preset classification object that does not exist in the current screened classification object set to the current screened classification object set, to obtain at least one current reference classification object set; and

using the current screened classification object set as the target classification object set until each current second classification performance is not better than the previous second classification performance.

6. The method according to any one of claims 1 to 5, wherein when the classification object dimension is the gene dimension, the target classification object set is a target drug resistance gene set;

accordingly, the acquiring a preset classification object set of a target drug corresponding to each of at least one classification object dimension comprises:

When each classification object dimension comprises the gene dimension and the mutation site dimension, based on the target drug resistance gene set corresponding to the gene dimension, performing a filtering operation on the reference drug resistance mutation information set to obtain a preset drug resistance mutation information set of the target drug corresponding to the mutation site dimension.

7. The method according to any one of claims 1 to 5, wherein, when the classification object dimension is the gene dimension, the target classification object set is a target drug resistance gene set, and when the classification object dimension is the mutation site dimension, the target classification object set is a target drug resistance mutation information set;

accordingly, the constructing in a drug resistance database, based on at least one target classification object set, a standard drug resistance mutation information set corresponding to the target drug comprises:

acquiring, when each classification object dimension comprises a gene dimension and a mutation site dimension having a parallel relationship, for each target drug resistance gene in the target drug resistance gene set corresponding to the gene dimension, a gene drug resistance mutation information set consisting of at least one piece of target drug resistance mutation information corresponding to the target drug resistance gene in the reference drug resistance mutation information set; and

based on at least one gene drug resistance mutation information set and the target drug resistance mutation information set corresponding to the mutation site dimension, constructing in the drug resistance database the standard drug resistance mutation information set corresponding to the target drug.

8. The method according to claim 1, wherein the method further comprises:

acquiring sample nucleic acid sequence data separately corresponding to each strain sample;

performing, for each strain sample, a mutation processing operation on the sample nucleic acid sequence data corresponding to the strain sample, to obtain a sample mutation information set of the strain sample;

screening, based on a drug resistance label separately corresponding to each strain sample, each sample mutation information set, to obtain at least two initial drug resistance mutation information sets; and

sequentially performing a union operation and a gene filtering operation on each initial drug resistance mutation information set, to obtain the reference drug resistance mutation information set corresponding to the target drug.

9. The method according to claim 1, wherein, when the classification object dimension is the gene dimension, the preset classification object set is a preset drug resistance gene set, and the object weight is a gene weight;

accordingly, the acquiring an object feature set of the target drug separately corresponding to each preset classification object in the preset classification object set of the classification object dimension comprises:

acquiring a sample mutation vector set of the target drug separately corresponding to each preset drug resistance gene in the preset drug resistance gene set of the gene dimension;

training, for each preset drug resistance gene, based on the sample mutation vector set corresponding to the preset drug resistance gene, a third initial model, to obtain a trained third target model;

determining, based on third classification performance corresponding to the third target model, a gene weight corresponding to the preset drug resistance gene; and

adding the sample mutation vector set and the gene weight corresponding to the preset drug resistance gene to an object feature set corresponding to the preset drug resistance gene.

10. The method according to claim 1, wherein when the classification object dimension is the mutation site dimension, the preset classification object set is a preset drug resistance mutation information set, the object weight is a mutation site weight, and each object mutation vector is a site mutation vector;

accordingly, the acquiring an object feature set of the target drug separately corresponding to each preset classification object in the preset classification object set of the classification object dimension comprises:

acquiring sample mutation features respectively corresponding to at least two strain samples, wherein each sample mutation feature comprises a site mutation vector of the strain sample corresponding to each piece of reference drug resistance mutation information in the reference drug resistance mutation information set;

training, based on each sample mutation feature, a fourth initial model, to obtain a trained fourth target model;

using, for each piece of reference drug resistance mutation information in the preset drug resistance mutation information set, a model weight corresponding to the reference drug resistance mutation information in the fourth target model as a mutation site weight; and

adding the sample mutation vector set and the mutation site weight corresponding to the reference drug resistance mutation information to an object feature set corresponding to the reference drug resistance mutation information.

11. A drug resistance testing method, **characterized by** comprising:

acquiring a mutation information set to be tested for a strain to be tested, wherein the mutation information set to be tested comprises at least one piece of mutation information to be tested;

acquiring in a drug resistance database a standard drug resistance mutation information set corresponding to a target drug, wherein the standard drug resistance mutation information set comprises at least one piece of standard drug resistance mutation information; and

determining, based on overlapping data corresponding to the mutation information set to be tested and the standard drug resistance mutation information set, a target drug resistance result of the strain to be tested against the target drug, wherein

the drug resistance database is obtained using the method for constructing a drug resistance database according to any one of claims 1 to 10.

12. The method according to claim 11, wherein the standard drug resistance mutation information set further comprises a mutation score separately corresponding to each piece of standard drug resistance mutation information, and the overlapping data comprises an overlap rate;

accordingly, the determining, based on overlapping data corresponding to the mutation information set to be tested and the standard drug resistance mutation information set, a target drug resistance result of the strain to be tested against the target drug comprises:

performing a union operation on the mutation information set to be tested and the standard drug resistance mutation information set, to obtain an overlapping mutation information set;

determining, based on the mutation score separately corresponding to each piece of standard drug resistance mutation information in the overlapping mutation information set, an overlap rate of the strain to be tested; and

determining, based on the overlap rate, the target drug resistance result of the strain to be tested against the target drug.

13. The method according to claim 11, wherein, before acquiring in a drug resistance database a standard drug resistance mutation information set corresponding to a target drug, the method further comprises:

acquiring sample mutation features respectively corresponding to at least two strain samples, wherein each sample mutation feature comprises a site mutation vector of the strain sample separately corresponding to each piece of reference drug resistance mutation information in the reference drug resistance mutation information set;

separately training, based on each sample mutation feature, at least two fifth initial models, to obtain at least two trained fifth target models;

determining, based on the mutation information set to be tested and the reference drug resistance mutation information set, a mutation feature to be tested corresponding to the strain to be tested;

separately inputting the mutation feature to be tested into the at least two fifth target models to obtain predicted drug resistance results respectively outputted by the respective fifth target models; and

using, when at least two predicted drug resistance results are the same, the predicted drug resistance results as the target drug resistance result of the strain to be tested against the target drug.

14. An apparatus for constructing a drug resistance database, **characterized by** comprising:

a preset classification object set acquisition module, configured to acquire a preset classification object set of a target drug corresponding to each of at least one classification object dimension;

an object feature set acquisition module, configured to acquire, for each classification object dimension, an object feature set of the target drug separately corresponding to each preset classification object in the preset classification object set of the classification object dimension;

a preset classification object set screening module, configured to screen, based on an object weight and a sample mutation vector set in each object feature set, the preset classification object set, to obtain a target classification object set; and

a drug resistance database construction module, configured to, based on at least one target classification object set, construct in a drug resistance database a standard drug resistance mutation information set corresponding to the target drug, wherein

each classification object dimension comprises a gene dimension and/or a mutation site dimension, the sample mutation vector set comprises at least two object mutation vectors, each object mutation vector comprises at least one mutation site identifier of a strain sample corresponding to the preset classification object, and the mutation site identifier represents whether reference drug resistance mutation information in a reference drug resistance mutation information set exists in a sample mutation information set corresponding to the strain sample.

15. A drug resistance testing apparatus, **characterized by** comprising:

> a mutation information set to be tested acquisition module, configured to acquire a mutation information set to be tested for a strain to be tested, wherein the mutation information set to be tested comprises at least one piece of mutation information to be tested;
> a standard drug resistance mutation information set acquisition module, configured to acquire in a drug resistance database a standard drug resistance mutation information set corresponding to a target drug, wherein the standard drug resistance mutation information set comprises at least one piece of standard drug resistance mutation information; and
> a target drug resistance result determination module, configured to determine, based on overlapping data corresponding to the mutation information set to be tested and the standard drug resistance mutation information set, a target drug resistance result of the strain to be tested against the target drug, wherein
> the drug resistance database is obtained using the method for constructing a drug resistance database according to any one of claims 1 to 10.

16. An electronic device, **characterized in that** the electronic device comprises:

> at least one processor; and
> a memory communicatively connected to the at least one processor, wherein
> the memory stores a computer program executable by the at least one processor, and the computer program is executed by the at least one processor so as to enable the at least one processor to perform the method for constructing a drug resistance database according to any one of claims 1 to 10, and/or the drug resistance testing method according to any one of claims 11 to 13.

17. A computer-readable storage medium, **characterized in that** the computer-readable storage medium stores computer instructions configured to, when executed by a processor, cause the processor to implement the method for constructing a drug resistance database according to any one of claims 1 to 10, and/or the drug resistance testing method according to any one of claims 11 to 13.

Acquire a preset classification object set of a target drug corresponding to each of at least one classification object dimension — S110

Acquire, for each classification object dimension, an object feature set of the target drug separately corresponding to each preset classification object in the preset classification object set of the classification object dimension — S120

Screen, based on an object weight and a sample mutation vector set in each object feature set, the preset classification object set, to obtain a target classification object set — S130

Based on at least one target classification object set, construct in a drug resistance database a standard drug resistance mutation information set corresponding to the target drug — S140

## FIG. 1

Sort_0={gene1 gene2 gene3 gene4}

Is Sort_i-1 empty? — Yes

No

Sort_i=Sort_i-1-gene i
volume_i=volume_i-1+gene i

Based on volume_i, train an SVM model, to obtain SVM_i

SVM_i>SVM_i-1? — Yes → i=i+1

No

volume_i=volume_i-1

Target_object=volume_i-1

## FIG. 2

For a gene dimension and based on a gene weight, iteratively add each preset drug resistance gene in a preset drug resistance gene set to a target drug resistance gene set, the basis for the addition being that adding the preset drug resistance gene increases the accuracy of a first target model by 1%

For a mutation site dimension and based on the target drug resistance gene set, filter a reference drug resistance mutation information set, to obtain a preset drug resistance mutation information set

Based on a mutation site weight, iteratively add each piece of reference drug resistance mutation information in the preset drug resistance mutation information set to a target drug resistance mutation information set, the basis for the addition being that adding the reference drug resistance mutation information increases the accuracy of the first target model by 1%

Use the target drug resistance mutation information set as a standard drug resistance mutation information set

Conventional drug resistance database

Merged drug resistance database

FIG. 3

Acquire sample nucleic acid sequence data separately corresponding to each strain sample — S210

Perform, for each strain sample, a mutation processing operation on the sample nucleic acid sequence data corresponding to the strain sample, to obtain a sample mutation information set of the strain sample — S220

Screen, based on a drug resistance label separately corresponding to each strain sample, each sample mutation information set, to obtain at least two initial drug resistance mutation information sets — S230

Sequentially perform a union operation and a gene filtering operation on each initial drug resistance mutation information set, to obtain a reference drug resistance mutation information set corresponding to a target drug — S240

Acquire a preset classification object set of the target drug corresponding to each of at least one classification object dimension — S250

Acquire, for each classification object dimension, an object feature set of the target drug separately corresponding to each preset classification object in the preset classification object set of the classification object dimension — S260

Screen, based on an object weight and a sample mutation vector set in each object feature set, the preset classification object set, to obtain a target classification object set — S270

Based on at least one target classification object set, construct in a drug resistance database a standard drug resistance mutation information set corresponding to the target drug — S280

# FIG. 4

Sort_0={gene1 gene2 gene3 gene4}

volume_i_1=volume_i-1+genei
...
volume_i_k=volume_i-1+genej

Based on volume_i_1-volume_i_k,
separately train SVM models to obtain
SVM_i_1-SVM_i_k

SVM_i_m>SVM_i-1?

No

Yes

i=i+1

SVM_i=SVM_i_m
volume_i=volume_i_m

Target_object=volume_i-1

## FIG. 5

Acquire a mutation information set to be tested for a strain to be tested — S310

Acquire in a drug resistance database a standard drug resistance mutation information set corresponding to a target drug — S320

Determine, based on overlapping data corresponding to the mutation information set to be tested and the standard drug resistance mutation information set, a target drug resistance result of the strain to be tested against the target drug — S330

## FIG. 6

FIG. 7

FIG. 8

**FIG. 9**

**FIG. 10**

EP 4 723 130 A1

FIG. 11

FIG. 12

## INTERNATIONAL SEARCH REPORT

| International application No. |
| --- |
| **PCT/CN2025/078835** |

| **A.** | **CLASSIFICATION OF SUBJECT MATTER** |
| --- | --- |

G16H70/40(2018.01)i

According to International Patent Classification (IPC) or to both national classification and IPC

| **B.** | **FIELDS SEARCHED** |
| --- | --- |

Minimum documentation searched (classification system followed by classification symbols)

IPC:G16H70/40

Documentation searched other than minimum documentation to the extent that such documents are included in the fields searched

Electronic data base consulted during the international search (name of data base and, where practicable, search terms used)

CNABS, CNTXT, DWPI, WOTXT, EPTXT, USTXT, CNKI, 万方, WANFANG, 读秀, DUXIU, PUBMED, ISI_Web of Science, Science Direct, STNext: 深圳市真迈生物科技有限公司, 陈燕香, 周乐天, 耐药, 数据库, 药物, 维度, 向量集, 突变, 基因, 突变点位标识, 对象集, 检测, 装置, 设备, 介质, resistance, database, drug, dimension, vector set, mutation, gene, mutation point identification, object set, detection, apparatus, device, medium

| **C.** | **DOCUMENTS CONSIDERED TO BE RELEVANT** |
| --- | --- |

| Category* | Citation of document, with indication, where appropriate, of the relevant passages | Relevant to claim No. |
| --- | --- | --- |
| A | WO 2021146953 A1 (BOE TECHNOLOGY GROUP CO., LTD.) 29 July 2021 (2021-07-29) claims 1-22 | 1-17 |
| A | CN 101421415 A (AVESTHA GENGRAINE TECHNOLOGIES PVT. LTD. et al.) 29 April 2009 (2009-04-29) claims 1-26 | 1-17 |
| A | CN 105593865 A (SIEMENS AG et al.) 18 May 2016 (2016-05-18) claims 1-15 | 1-17 |
| A | CN 115810391 A (CAPITALBIO CORP.) 17 March 2023 (2023-03-17) claims 1-10 | 1-17 |
| A | KR 20200064469 A (KOREAN NATIONAL TUBERCULOSIS ASSOCIATION) 08 June 2020 (2020-06-08) claims 1-7 | 1-17 |
| A | WO 2016049929 A1 (BGI TIANJIN et al.) 07 April 2016 (2016-04-07) claims 1-50 | 1-17 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **13 June 2025** | **19 June 2025** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2025/078835**

**C. DOCUMENTS CONSIDERED TO BE RELEVANT**

| Category* | Citation of document, with indication, where appropriate, of the relevant passages | Relevant to claim No. |
|---|---|---|
| A | CN 114566209 A (SICHUAN UNIVERSITY) 31 May 2022 (2022-05-31) claims 1-20 | 1-17 |

Form PCT/ISA/210 (second sheet) (July 2022)

**INTERNATIONAL SEARCH REPORT**
Information on patent family members

International application No.

**PCT/CN2025/078835**

| Patent document cited in search report | | | Publication date (day/month/year) | Patent family member(s) | | | Publication date (day/month/year) |
|---|---|---|---|---|---|---|---|
| WO | 2021146953 | A1 | 29 July 2021 | None | | | |
| CN | 101421415 | A | 29 April 2009 | WO | 2006008575 | A2 | 26 January 2006 |
| | | | | WO | 2006008575 | A9 | 12 August 2010 |
| | | | | EP | 1789577 | A2 | 30 May 2007 |
| | | | | EP | 1789577 | A4 | 17 November 2010 |
| CN | 105593865 | A | 18 May 2016 | WO | 2015007487 | A1 | 22 January 2015 |
| | | | | US | 2016162635 | A1 | 09 June 2016 |
| CN | 115810391 | A | 17 March 2023 | None | | | |
| KR | 20200064469 | A | 08 June 2020 | None | | | |
| WO | 2016049929 | A1 | 07 April 2016 | None | | | |
| CN | 114566209 | A | 31 May 2022 | None | | | |

Form PCT/ISA/210 (patent family annex) (July 2022)